# EUROPEAN PATENT SPECIFICATION

(11) **EP 3 604 524 B1**
(45) Date of publication and mention of the grant of the patent: **06.10.2021**
(21) Application number: 17901979.9
(22) Date of filing: 02.08.2017
(51) Int. Cl.: C12N 15/10, C12N 15/66

(54) **NEW TECHNIQUE FOR GENOMIC LARGE FRAGMENT DIRECT CLONING AND DNA MULTI-MOLECULAR ASSEMBLY**
NEUE TECHNIK FÜR DIREKTE KLONIERUNG GENOMISCHER GROSSFRAGMENTE UND DNA-MULTIMOLEKULARE ANORDNUNG
NOUVELLE TECHNIQUE DE CLONAGE DIRECT DE GRANDS FRAGMENTS GÉNOMIQUES ET ENSEMBLE MULTI-MOLÉCULAIRE D'ADN

(30) Priority: 23.03.2017 CN 201710177676
(43) Date of publication of application: 05.02.2020
(73) Proprietor: Shandong University, Qingdao, Shandong 266237 (CN); Technische Universität Dresden, 01069 Dresden (DE)
(72) Inventor: ZHANG, Youming, Jinan Shandong 250100 (CN); WANG, Hailong, Jinan Shandong 250100 (CN); FU, Jun, Jinan Shandong 250100 (CN); STEWART, Adrian Francis, 01307 Dresden (DE)
(74) Representative: Cabinet Chaillot
(86) International application number: PCT/CN2017/000483
(87) International publication number: WO 2018/170614

(56) References cited:
- WO-A1-98/38297
- WO-A2-2007/021944
- WO-A2-2007/032837
- WO-A2-2016/033315
- CN-A- 102 634 534
- CN-A- 103 068 995
- CN-A- 106 995 813
- US-A1- 2015 072 381
- JUN FU ET AL: "Full-length RecE enhances linear-linear homologous recombination and facilitates direct cloning for bioprospecting", NATURE BIOTECHNOLOGY, vol. 30, no. 5, 29 April 2012 (2012-04-29) , pages 440-446, XP055678843, us ISSN: 1087-0156, DOI: 10.1038/nbt.2183
- KOLODNER R ET AL: "HOMOLOGOUS PAIRING PROTEINS ENCODED BY THE ESCHERICHIA COLI RECE AND RECT GENES", MOLECULAR MICROBIOLOGY, WILEY-BLACKWELL PUBLISHING LTD, GB, vol. 11, no. 1, 1 January 1994 (1994-01-01), pages 23-30, XP002064301, ISSN: 0950-382X, DOI: 10.1111/J.1365-2958.1994.TB00286.X
- LI, M.Z.: "Harnessing homologous recombination in vitro to generate recombinant DNA via SLIC", NATURE METHODS, vol. 4, no. 3, 11 February 2007 (2007-02-11), pages 251-256, XP002564746,
- WANG, H.: "RecET direct cloning and Red recombineering of biosynthetic gene clusters, large operons or single genes for heterologous expression", NATURE PROTOCOLS, vol. 11, no. 7, 2 June 2016 (2016-06-02), pages 1175-1190, XP055607964,
- Wang, Junping et al.,: "The Application of Red/ET Recombination to High Efficient Gene-targeting Vector Construction", Hereditas, vol. 27, no. 6, 31 December 2005 (2005-12-31), pages 953-958, XP009516860, ISSN: 0253-9772
- Zhu, Ying et al.,: "Progress of in vivo direct Cloning of Large DNA Fragments", China Biotechnology, vol. 34, no. 4, 31 December 2014 (2014-12-31), pages 95-100, XP055636116, DOI: 10.13523/j.cb.20140415

## Description

The disclosure relates to a method of nucleic acid cloning and assembly, and more particularly to a method of deoxyribonucleic acid (DNA) cloning and assembly.

DNA cloning is a core technique used in molecular biology and biotechnology for gene function studies. With the progress of DNA sequencing technology and the decline in sequencing costs, genome-wide sequencing is becoming easier. Researchers have found that there are abundant untapped resources in the genome. Short DNA fragments are easily obtained by PCR or chemical synthesis, but the conventional method of cloning DNA fragments larger than 10-kb depends on the construction and screening of DNA libraries. Conventional methods of library construction and screening are complicated, time-consuming and laborious, and the target DNA fragments are often located on several different regions. Researchers subclone the fragments, remove redundant sequences or integrate them into a complete biosynthetic pathway for investigating gene function, so the conventional methods of library construction and screening can no longer meet the needs of the times, and researchers urgently need simple, efficient and fast genomic DNA cloning and modification techniques. With the progress techniques used in synthetic biology, large DNA fragments larger than 10-kb can also be assembled from selected small fragments using Gibson assembly in vitro¹ or DNA assembler in vivo². However, in the above DNA assembly process, small fragments are only prepared by PCR or chemical synthesis, so it is easy to introduce random mutations to cause inconvenience in subsequent gene function studies. The above methods require DNA fragments having a high purity and concentration, and cannot be used for directly assembling a target DNA fragment from a mixture of enzymatic genomic DNA fragments.

A specific DNA sequence from genomic DNA is cloned directly into the vector, referred to herein as direct cloning. The recombinant proteins RecE and RecT of Rac phage are capable of mediating efficiently homologous recombination between linear DNA molecules in the cells *of E. coli,* also known as linear-linear recombination. RecE is a 5' to 3' exonuclease, and RecT is a single-stranded DNA (ssDNA)-annealing protein, and protein-protein interaction between RecE and RecT is required for linear-linear recombination, in particular, the linear-linear recombination efficiency of RecET combined action is 1000 times that of the single effect. RecET direct cloning technique can directly capture genomic DNA fragments larger than 10-kb into an expression vector, and can also assemble 2-5 DNA fragments. Suitable restriction enzyme sites can be found in the genome to release the target DNA fragment, and then the genomic DNA and the linear vector digested with restriction enzymes are electroporated into *E. coli cell* expressing RecET recombinase. The target DNA fragment and the linear vector in the cells are homologously recombined by homologous boxes at both ends to form a circular plasmid, and finally the colonies containing the recombinant DNA molecule can be obtained by antibiotic screening and restriction analysis. After preparing the linear vector and the genomic DNA, it takes only 3 days to complete the direct cloning of the target DNA fragment. At present, this technique has been widely used in the cloning and heterologous expression studies of bacterial natural product biosynthesis pathways. For example, ten10-52-kb polyketide synthases (PKS)/non-ribosomal polypeptide synthetase (NRPS) gene clusters³ derived from *Photorhabdus luminescens,* 12-kb sevadicin gene clusters⁴ derived from *Paenibacillus larvae,* 19-kb syringolin gene clusters⁵ derived from *Pseudomonas syringae,* 25-kb glidobactin gene clusters⁶ derived from *Burkholderia* DSM7029, 50-kb colibactin gene clusters⁷ derived from *E. coli* Nissle 1917.

The advantages of direct cloning drive many of the relevant research in the field compared to library construction and screening. Larionov et al. established a TAR cloning technique⁸ that relies on a yeast recombination system, and then they combine TAR with Cas9 cleavage to increase the efficiency of TAR cloning for easier application^{9,10}. The recently established Cas9-Assisted Targeting of Chromosome Segments (CATCH) technology splices large DNA fragments into BAC¹¹ using Cas9 in vitro cleavage using and Gibson assembly. This method is currently only applied to prokaryotic genomes, and PCR pre-screening of colonies is required prior to the restriction enzyme digestion of recombinant DNA. To the best of the inventors' knowledge, all currently known direct cloning methods, including the above two methods, are limited to some experts to manipulate.

Although RecET direct cloning is convenient for cloning DNA fragments below 50-kb from bacterial genome^{3-6,12}, it is difficult to clone larger DNA fragments from bacterial genome or clone DNA fragments from mammalian genome. The reason is that RecET direct cloning technique relies on the intracellular expression of RecET recombinase, and homologous recombination occurs only when the cloning vector and the target DNA fragment simultaneously enter *E. coli* cells and meet, which leads to the following defects: (1) Limit the size of genomic DNA fragments. For example, it is not possible to clone the DNA fragments larger than 50-kb from bacterial genome or cloning the genome fragments larger than 10-kb from mammals (such as mice and humans). This is because the concentration of a certain DNA fragment in the genome is very low, which results in a small probability that the target DNA fragment and the cloning vector enter cells at the same time during a transformation. Moreover, due to the influence of physical shear force during the preparation of genomic DNA, the larger the target DNA fragment, the lower the concentration in the genome, which results in the probability that the target DNA fragment enters the cells together with the cloning vector is lower. The genomes of mice (2800 Mb) and humans (3200 Mb) are nearly three orders of magnitude larger than the bacterial genome (5 Mb), so the concentration of genomic DNA fragments of the same size are much lower in mammalian genome than bacteria. Therefore, the probability that the target DNA fragment enters *E. coli* cells simultaneously with the cloning vector is much lower when directly cloning a genomic DNA fragment of mammalian. (2) Affected by the number of DNA fragments, during the assembly of multiple fragments, the assembly efficiency is drastically reduced as the number of DNA fragments exceeds four. At present, RecET direct cloning technique only assembles up to five DNA fragments. For multiple fragment assembly, the more the number of DNA fragments, the lower the possibility that they enter cells together, and the lower the assembly efficiency. Therefore, the field particularly needs an easy-to-operate and widely used direct cloning technique that is not limited by the size of resultant DNA fragment and the genome complexity.

To solve the technical problem of directly cloning large DNA fragments from the genome, the inventors first cleave the genomic DNA and the cloning vector with exonuclease in vitro, the reaction product in vitro was then homologously recombined in the presence of RecET recombinase to establish the ExoCET cloning technique. ExoCET technique can clone DNA fragments larger than 100-kb directly from bacterial genome, and clone DNA fragments larger than 50-kb from mammalian cells and human blood. ExoCET technique is also capable of efficiently assembling at least twenty DNA fragments to form a complete plasmid.

Like the RecET direct cloning technique, ExoCET has a comparative advantage over PCR in that it has high fidelity and does not destroy the DNA haplotype, and the target DNA is directly cloned into a plasmid vector to facilitate expression studies. In addition, ExoCET is more efficient than Gibson assembly because Gibson relies on circular DNA molecules produced by in vitro assembly. Moreover, due to the cyclization of the empty carrier itself, Gibson assembly may produce a very serious background in the process of direct cloning, which can be the reason why Jiang et al¹¹ perform PCR pre-screening before identifying the recombinant DNA; it is also the reason why Zhou et al¹³ need to remove the genomic DNA fragment of 20-kb or smaller generated by restriction digestion and agarose gel electrophoresis, when using Gibson assembly to clone conglobatin gene cluster from *Streptomyces conglobatus* genome.

ExoCET can also be used to directly clone DNA fragments from mammalian genomes such as blood, disease-associated cell lines, etc. to facilitate haplotype studies of single-nucleotide polymorphisms (SNPs) and to rapidly construct haplotype isogenic targeting (HIT) vector for nuclease-mediated human stem cell targeting. The importance of human stem cells isolated from patients, cord blood or somatic cells reprogramming in biomedical research has received more and more attention, and the research on the precise modification of stem cell genome has also received widespread attention. Modifying the human genome is more challenging than modifying the laboratory mice genome due to the complex human genetic diversity. The importance of isogenicity (sequence similarity) for homologous recombination was realized many years ago when people use mouse embryonic stem cells for gene targeting¹⁴. However, the effect of sequence mismatch on homologous recombination has not been well studied. For example, how much recombination efficiency does a single mismatch (a SNP or an indel) reduce? How does the distance of the mismatched site from the recombination site affect the recombination efficiency? How do multiple mismatches affect recombination efficiency? These above issues have not yet been clarified. In any case the use of identical sequences in gene targeting is clearly highly recommended, so ExoCET is an effective technique to quickly obtain ideal homology boxes. Unlike the method of amplifying homology boxes from the genome by PCR, ExoCET is not limited by fragment sizes, does not introduce mutations, and is capable of maintaining a DNA haplotype. Furthermore, the ends of the homology boxes can also be selected according to the forms of genotyping (such as Southern blotting or ligation PCR), optimizing the length of the homology boxes. ExoCET, therefore, offers advantages for individualized genomic surgery, especially when combined with CRISPR/Cas9¹⁵. ExoCET can also be used as the most reliable method for genotyping a modified genome, while Southern blotting and ligation PCR have false positive signals.

Fu, J et al. Nature Biotech (2012) 30(5):440-446; US 2015/072381; and Kolodner R et al. Mol Microbiol (1994) 11(1):23-30 disclose methods based on RecET homologous recombination.

ExoCET is capable of selectively capturing large DNA fragments from complex genomes, making it becomes a means of disease diagnosis and pathology testing. For example, capturing DNA sequences directly from individualized medicine or isolating DNA viruses from patient samples. ExoCET will be widely used in functional genomics and comparative genomics research, especially for direct cloning of prokaryotic synthesis pathways or assembling multiple DNA molecules for synthetic biology.

The invention is defined by the appendant claims.

In one aspect, the disclosure provides a method of homologous recombination, the method comprising treating two or more target nucleic acid molecules with a first exonuclease, recombining the two or more target nucleic acid molecules after being treated in the presence of a second exonuclease and an annealing protein, where recombined target nucleic acid molecules share at least one homologous sequence.

In another aspect, the disclosure provides a method of homologous recombination, the method comprising treating a first nucleic acid molecule and a second nucleic acid molecule with a first exonuclease, recombining the first nucleic acid molecule and the second nucleic acid molecule after being treated in the presence of a second exonuclease and an annealing protein, where the first nucleic acid molecule and the second nucleic acid molecule share at least one homologous sequence.

In still another aspect, the disclosure provides a method of assembling a linear nucleic acid molecule, the method comprising treating two or more nucleic acid molecules with a first exonuclease, recombining the two or more nucleic acid molecules after being treated in the presence of a second exonuclease and an annealing protein, where each nucleic acid molecule shares at least one homologous sequence with an adjacent nucleic acid molecule in a resulting assembly product.

Also, the disclosure provides a method of cloning genomic DNA, the method comprising treating a genomic DNA fragment mixture and a linear cloning vector with a first exonuclease, recombining the genomic DNA fragment mixture and the linear cloning vector after being treated in the presence of a second exonuclease and an annealing protein, where the linear cloning vector shares at least one homologous sequence with the target DNA fragment of the mixture of genomic DNA fragments.

The at least one homologous sequence can be located inside or at one end of the two or more target nucleic acid molecules, particularly, at least one homologous sequence at one end of a target nucleic acid molecule, and more particularly, all the homologous sequences at one end of the target nucleic acid molecules.

The at least one homologous sequence has a length of at least 6, at least 10, at least 20, at least 25, at least 30, at least 40, at least 50, at least 60, at least 70, at least 80 nucleotides, particularly 25, 40 or 80 nucleotides, and more particularly 80 nucleotides.

The first exonuclease can be a 5' to 3' exonuclease or a 3' to 5' exonuclease, particularly T4 DNA polymerase, Klenow fragment of DNA polymerase I, T5 exonuclease, T7 exonuclease, and more particularly T4 DNA polymerase or T5 exonuclease.

Treating in the presence of the first exonuclease comprises in vitro joining. The in vitro joining can join two or more target nucleic acid molecules or the first nucleic acid molecule to the second nucleic acid molecule, or join the treated linear cloning vector to the target DNA fragment of a mixture of genomic DNA fragments.

Treating in the presence of the first exonuclease comprises enzyme digestion and annealing; the enzyme digestion of different nucleic acid molecules can be performed separately or simultaneously, such as in a mixture of samples.

Treating in the presence of the first exonuclease further comprises adding a DNA polymerase, dNTP and DNA ligase.

Treating in the presence of the first exonuclease further comprises the addition of a DNA polymerase having 3' to 5' exonuclease activity.

Treating in the presence of the first exonuclease excludes the addition of dNTPs.

Treating in the presence of the first exonuclease comprises T4 DNA polymerase treatment or Gibson assembly.

The second exonuclease is RecE, and particularly, the RecE is a recombinant expression product.

The single-stranded annealing protein includes RecA, RAD51, Redβ, RecT, Pluβ or RAD52, and particularly, the annealing protein is RecT, more particularly, the RecT is a recombinant expression product.

The annealing protein is RecT, particularly, the RecT is a recombinant expression product.

The homologous recombination is carried out in vitro or in a host cell.

The host cell can be a yeast cell, particularly the yeast cell is a *Saccharomyces cerevisiae* cell; or a bacterial cell, particularly the bacterial cell is *Bacillus subtilis* or *Escherichia coli.*

The host cell expresses an exonuclease, particularly a second exonuclease and an annealing protein.

The host cell expresses an exonuclease, an annealing protein, and Redγ. Particularly, the host cell further expresses RecA, more particularly, the host cell expresses RecE, RecT, Redy, and RecA.

The host cell is *E. coli* cell expressing full length RecE and/or RecT, particularly, the host cell is *E. coli* cell expressing full length RecE, RecT and Redy, more particularly the host cell is *E. coli* cell expressing full length RecE, RecT, Redγ and RecA.

The host cell is *E. coli* cell expressing truncated RecE and RecT.

The host cell is *E. coli* cell expressing Redα and Redβ.

The host cell expresses an exonuclease on a plasmid vector and/or chromosome, particularly, the second exonuclease, the annealing protein, Redγ and/or RecA, particularly, expressing by a plasmid vector, and more particularly, expressing by a plasmid vector and a chromosome simultaneously.

The target nucleic acid molecule or the target DNA fragment is a linear DNA segment selected from a DNA fragment digested by endonuclease, a DNA fragment amplified by PCR, a genomic DNA fragment, a member of cDNA library, a fragment derived from bacterial artificial chromosomes (BACs), and a fragment of cloning vectors.

The endonuclease can be a restriction enzyme or a programmable endonuclease, such as Cas9.

The number of the target nucleic acid molecules or DNA fragments is 2, 3, 4, 5, 6, 7, 8, 9, 10, 11, 12, 13, 14, 15, 16, 17, 18, 19, 20 or more.

The target nucleic acid molecule comprises a sequence of 0.5 kb or longer (e.g., 1-kb or longer, 2.5-kb or longer, 4-kb or longer, 5-kb or longer, 7.5-kb or longer, 10-kb or longer, 15-kb or longer, 20-kb or longer, 25-kb or longer, 40-kb or longer, 50-kb or longer, 75-kb or longer or 100-kb or longer).

The two or more target nucleic acid molecules, the first target nucleic acid molecule, and the second target nucleic acid molecule or target DNA fragments comprise one or more PCR amplified DNA fragments, genomic DNA fragments, cDNA library members, and/or a fragment derived from BAC.

The first exonuclease has 3' to 5' exonuclease activity, and particularly, is T4 DNA polymerase.

Treating with an exonuclease is carried out in vitro in the absence of dNTPs.

The second exonuclease is a full length RecE.

The annealing protein is RecT.

The homologous recombination is carried out in a bacterial host cell expressing the full-length RecE and RecT, particularly in *Escherichia coli.*

The two or more target nucleic acid molecules comprise one or more PCR amplified DNA fragments, genomic DNA fragments, cDNA library members, and/or a fragment derived from BAC, linear plasmid and/or cloning vector fragment, and particularly, three or more linear plasmids and/or cloning vector fragments.

The first exonuclease comprises a Gibson assembly.

The second exonuclease is full length RecE.

The annealing protein is RecT.

The homologous recombination is carried out in a bacterial host cell expressing the full length RecE and RecT, particularly in *E. coli.*

The two or more target nucleic acid molecules include three or more PCR amplified DNA fragments, genomic DNA fragments, cDNA library members, and/or a fragment derived from BAC, linear plasmids and/or cloning vector fragments, particularly three or more linear plasmids and/or cloning vector fragments.

The first exonuclease comprises a Gibson assembly.

The second exonuclease is full length RecE.

The annealing protein is RecT.

The homologous recombination is carried out in a bacterial host cell expressing the full length RecE and RecT, particularly in *E. coli.*

Also provided is a kit comprising the first exonuclease and the second exonuclease described in the preceding method, or a nucleic acid encoding the first exonuclease and the second exonuclease described in the preceding method.

A kit comprises the first exonuclease and the second exonuclease described in the preceding method, or a nucleic acid encoding the first exonuclease and the second exonuclease. Particularly, the kit further comprises a host cell expressing the second exonuclease, particularly, the host cell expresses an exonuclease, an annealing protein and Redy, particularly, the host cell also expresses RecA, more particularly, the host cell expresses RecE, RecT, Red γ and RecA, the host cell can be a yeast cell, particularly the yeast cell is a *Saccharomyces cerevisiae* cell; or a bacterial cell, particularly the bacterial cell is *Bacillus subtilis* or *Escherichia coli,* The host cell expresses an exonuclease, an annealing protein, Redγ and/or RecA on a plasmid vector and/or chromosome simultaneously, particularly, expressed by a plasmid vector, the more particularly, expressed by a plasmid vector or chromosome, further particularly, the kit may further comprise one or more pre-prepared linear vectors.

The first exonuclease is a DNA polymerase having 3' to 5' exonuclease activity, such as T4 DNA polymerase, Klenow fragments of DNA polymerase I, T5 exonuclease or T7 exonuclease, the second exonuclease is full length RecE.

The kit further comprises a host cell expressing a second exonuclease, particularly, the host cell comprises a nucleic acid encoding of full length RecE, RecT, Redγ and RecA.

The kit further comprises one or more pre-prepared linear vectors.

Use of the aforesaid method or kit in construction of a targeting vector.

Use of the aforesaid method or kit in genotyping of mammalian cells.

Use of the aforesaid method or kit in DNA synthesis.

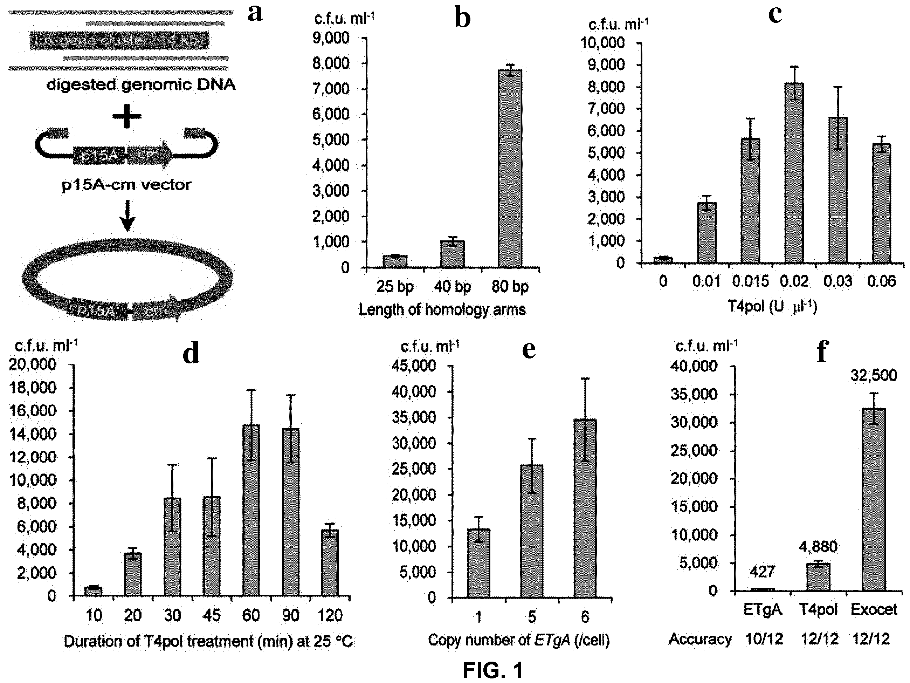

FIG. **1** shows concerted action of in vitro assembly and full length RecE/RecT improves the efficiency of direct cloning. (a): A schematic diagram of direct cloning of the 14-kb *lux* gene cluster from *P. phosphoreum* ANT-2200 genome. The p15A-cm vector and the target genomic DNA fragment have the same sequence at the very end. (b): Longer homology arms increase the cloning efficiency of ExoCET. Linear vectors with 25-bp, 40-bp or 80-bp homology boxes were mixed with genomic DNA, respectively, and the reaction was carried out for 20 minutes at 25°C with 0.02 U µL⁻¹ of T4pol before annealing and conversion to arabinose-induced *E. coli* GB05-dir. Error bars, s.d.; n = 3. (c): Optimizing the concentration of T4pol. The linear vector with 80-bp homology boxes and the genomic DNA was treated in the same manner as in (b), except for the different concentration of T4pol. (d): Effect of incubation time of T4pol on cloning efficiency. As with (c), 0.02 U µL⁻¹ of T4pol was used, but with different incubation time. (e): Higher copy number of ETgA increases ExoCET cloning efficiency. As with (d), the incubation time was 1 h, and then the in vitro assembled products were separately electroporated into arabinose-induced *E. coli* GB05-dir (with a copy of ETgA on the chromosome), containing GB2005 (with -5 copies of ETgA on pSC101 plasmid) of pSC101-BAD- ETgA-tet or containing GB05-dir (with -6 copies of ETgA) of pSC101-BAD-ETgA-tet. (f): ExoCET increases the efficiency of direct cloning. As with (e), GB05-dir (ExoCET) containing pSC101-BAD-ETgA-tet was used, or T4pol treatment (ETgA) was not used, or arabinose induction (T4pol) was not used.

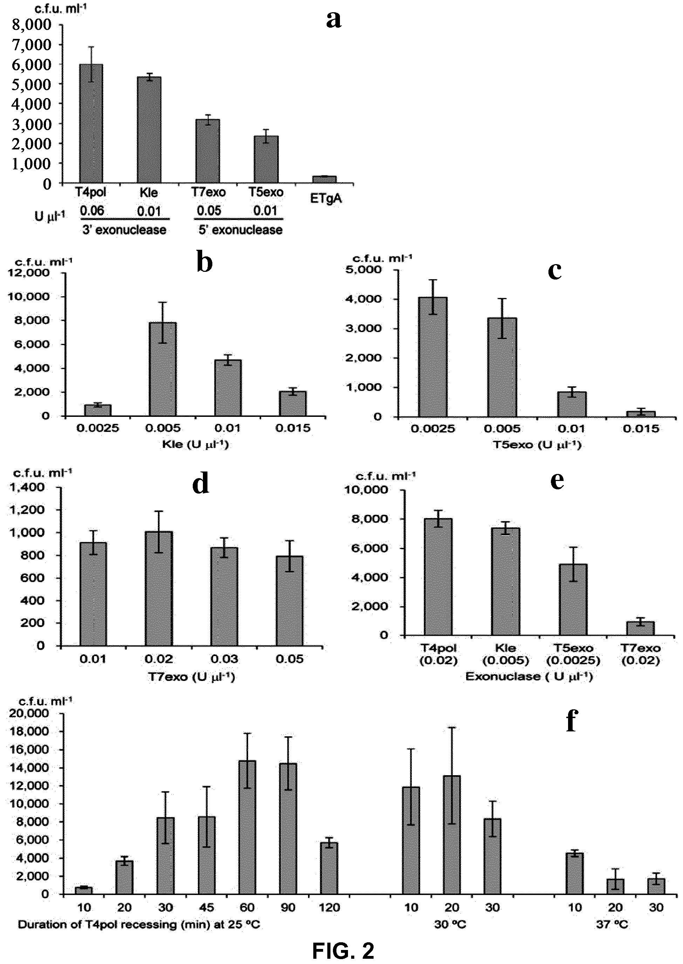

FIG. **2** shows effect of different exonucleases on direct cloning of the *lux gene* cluster. The p15A-cm vector and the *P. phosphoreum* genomic DNA were treated with exonuclease, annealed and then electroporated into arabinose-induced *E. coli* GB05-dir. (a): Initial detection of different exonucleases. (b-d): Optimizing the concentrations of Kle, T5exo and T7exo. (e): Comparison of cloning efficiency after 20 min digestion with T4pol, Kle, T5exo and T7exo at optimal concentrations. (f): Optimizing the digestion temperature and time of T4pol (0.02 U µL⁻¹). Error bars, s.d.; n = 3.

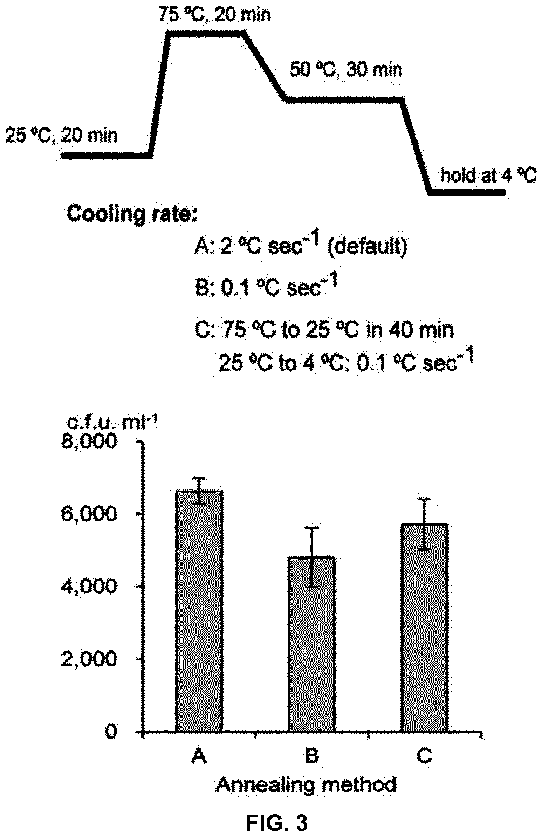

FIG. **3** shows effect of annealing rate on direct cloning of the *lux* gene clusters. Error bars, s.d.; n = 3. The p15A-cm vector and the *P. phosphoreum* genomic DNA were digested with 0.02 U µL⁻¹ T4pol and then annealed in different methods (A, B, C) and finally electroporated into arabinose-induced *E. coli* GB05-dir.

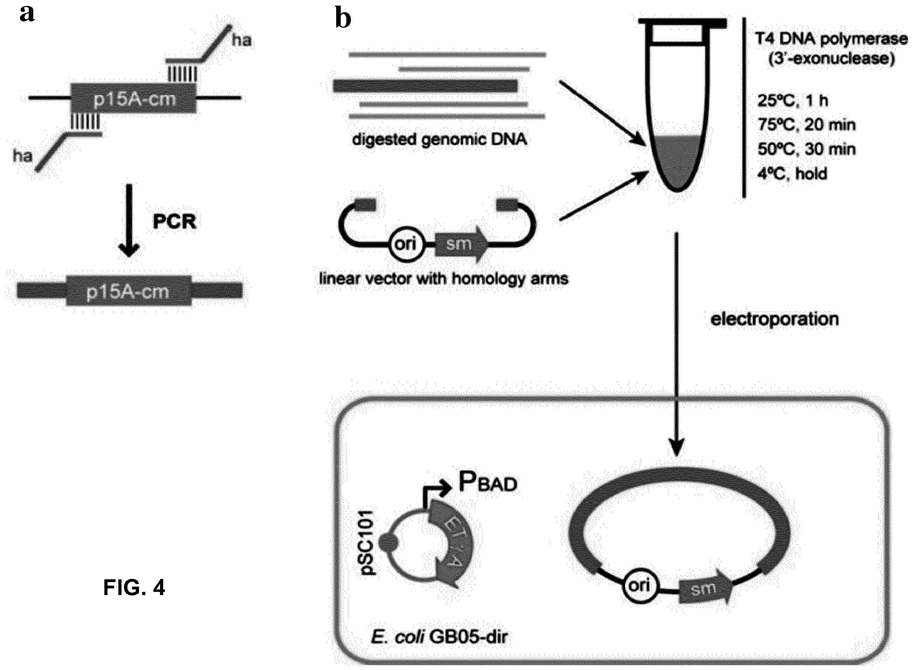

FIG. **4** is a flowchart of preparation of linear cloning vector and ExoCET direct cloning technique. (a): The p15A-cm vector was prepared by PCR amplification and the primers employed carry a homology box of 80 nucleotides. (b): The standard strategy for ExoCET direct cloning. The in vitro assembled product was transformed into arabinose-induced GB05-dir containing pSC101-BAD-ETgA-tet, and the correct recombinant was obtained by antibiotic screening.

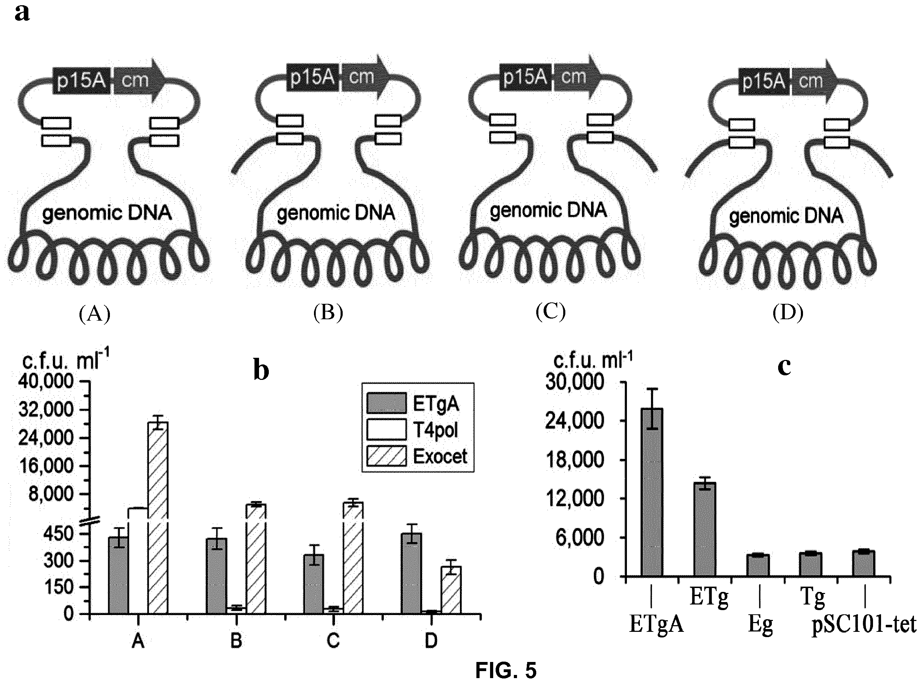

FIG. **5** (a): The position of the 80-bp homology boxes between the p15A-cm and the 14-kb *lux* genomic DNA fragments: (A) Both homology boxes were at the very end; (B, C) one was located 1 kb from the end and the other was at the very end; (D) Both were located 1-kb from the end. The reaction conditions were the same as in (1f). (b): The number of colonies was obtained by ETgA, T4pol or ExoCET combining with the above four homology boxes. (c): Using ExoCET and terminal homology boxes in GB2005, the direct cloning efficiency of 14-kb *lux gene* cluster was obtained by the different combinations of recombinant proteins expressed by pSC101 plasmid: ETg-no express RecA; Eg-no express RecA and RecT; Tg-no express RecA and RecE; pSC101-tet-empty vector. Error bars, s.d.; n = 3.

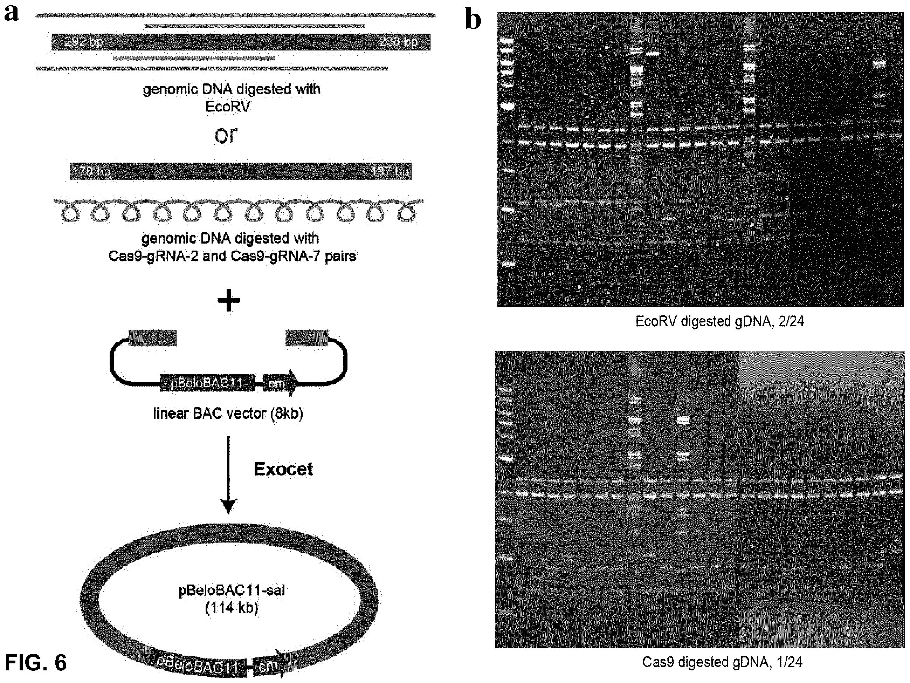

FIG. **6** shows direct cloning of the 106-kb salinomycin gene cluster using the *Streptomyces albus* genomic DNA digested with EcoRV or Cas9. (a): Under the action of ExoCET, the salinomycin gene cluster was cloned from genomic DNA digested with EcoRV or Cas9 gRNA2/Cas9-gRNA7 and was then inserted into the pBeloBAC11 vector. The homology box (blue) was first inserted into the BAC vector and the BAC vector was linearized by BamHI digestion to be a direct cloning vector. The length of the homology box was marked at the end of the genomic DNA fragment. (b): Pvull restriction analysis of recombinant DNA, the correct clones were marked by arrows.

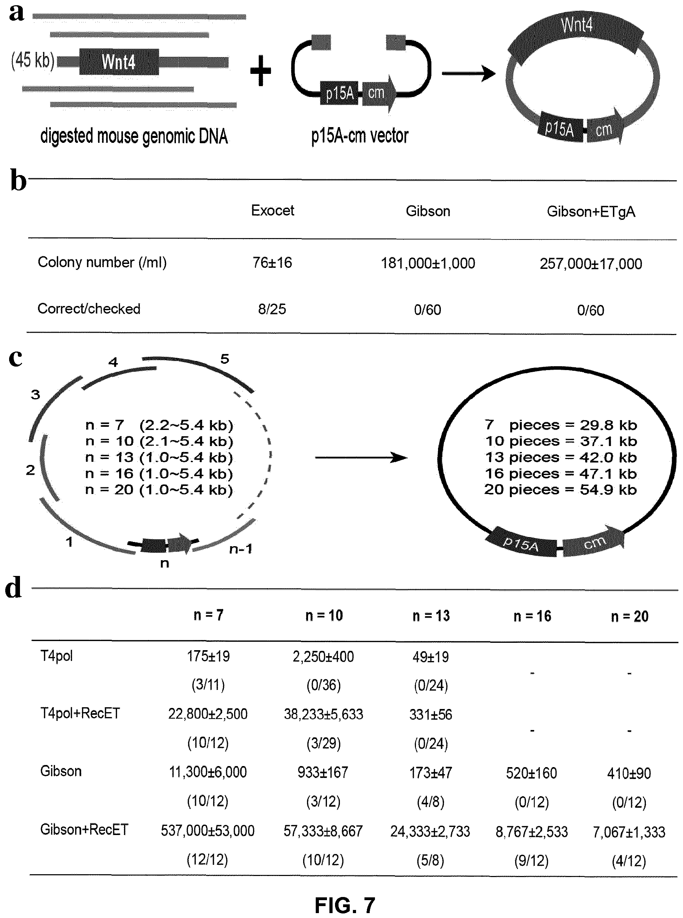

FIG. **7** shows comparison of the efficiency of ExoCET and Gibson assembly. (a): A schematic diagram of direct cloning of a 45-kb DNA fragment containing the Wnt4 gene from the mouse genome using the terminal homology boxes. (b): Number of colonies obtained by the ExoCET and Gibson methods. The mouse genomic DNA digested with p15A-cm and Swal were treated with ExoCET and Gibson, respectively, and then transformed into the GB05 containing pSC101-BAD-ETgA-tet-dir, whether arabinose-induction (ExoCET or Gibson+ETgA) or non-induction (T4pol or Gibson). (c): A schematic diagram of assembling multiple DNA fragments into plasmids by T4pol, ExoCET, Gibson and Gibson+ETgA. The size range of the DNA fragment was 1.0-kb to 5.4-kb, the size range of the assembled p5A plasmid was 29.8-kb to 54.9-kb and the plasmids were resistant to chloramphenicol (cm). (d): The number of clones and the correct rate obtained by the experiment of multiple fragment assembly. The in vitro assembled product was transformed into GB05-dir containing pSC101-BAD-ETgA-tet, whether arabinose-induction (ExoCET or Gibson+ETgA) or non-induction (T4pol or Gibson).

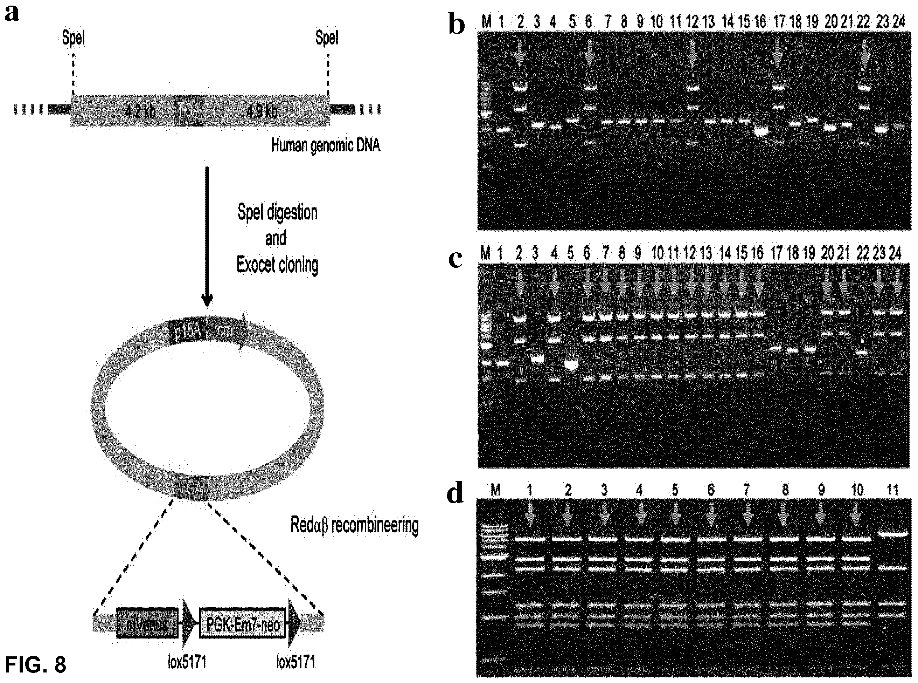

FIG. **8** shows construction of HIT (haplotype isogenic targeting) vectors for DPY30 using mammalian genomic DNA. (a): A schematic diagram of the cloning of DPY30 stop codon using human genomic DNA digested with Spel. After the direct cloning was completed, the C-terminus of DPY30 was labeled by the mVenus element ^{16,17} of the Redαβ recombineering. (b): Employing the genomic DNA isolated from human blood as a template, the recombinant DNA directly cloned by ExoCET was subjected to EcoRI digestion analysis. (c): EcoRI digestion analysis of the recombinant DNA obtained by direct cloning through ExoCET and genomic DNA isolated from human embryonic kidney 293T cells. (d): The Pvull digestion analysis of recombinant DNA obtained after inserting the mVenus element by Redαβ recombineering. All clones obtained were correct, with lane 11 being the control. The correct clone was marked by arrows.

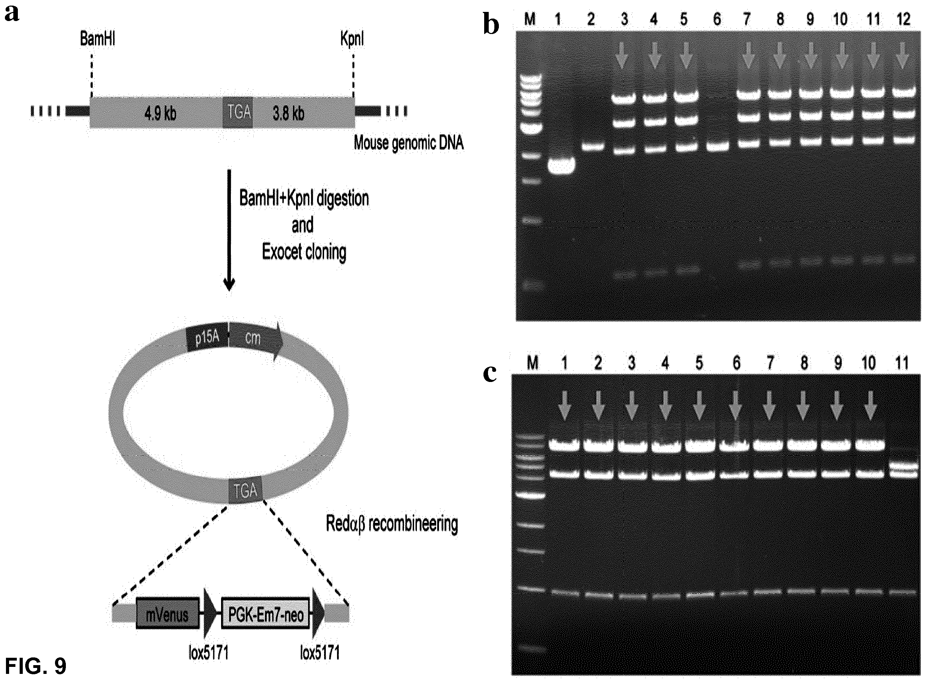

FIG. **9** shows construction of HIT (haplotype isogenic targeting) vectors for Dpy30 using mouse genomic DNA. (a): A schematic diagram of the cloning of Dpy30 stop codon using mouse genomic DNA digested with BamHI+KpnI. Once directly cloned, the C-terminus of DPY30 was tagged with a mVenus cassette^{16,17} using Redαβ recombineering. (b): EcoRI digestion analysis for the recombinant DNA obtained by direct cloning through ExoCET and genomic DNA isolated from mouse melanoma B16 cells. (c): Nhel digestion analysis of the recombinant DNA obtained by inserting the mVenus element of the Redαβ recombinant engineering. All clones obtained were correct, with lane 11 being the control. The correct clone was marked by arrows.

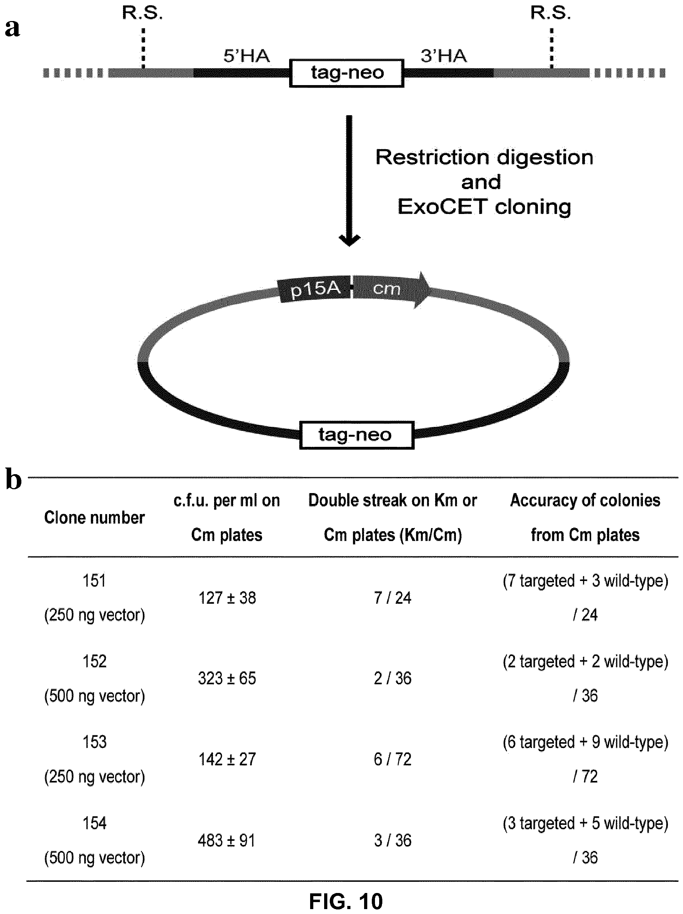

FIG. **10** shows genotyping of mammalian cells using ExoCET. (a): A schematic diagram of genotyping using ExoCET. Restriction sites were located upstream and downstream of the targeting element, respectively. (b): Genotyping of mouse embryonic stem cells targeted by Kmt2d-AID-neo with kanamycin resistance using ExoCET. A DNA fragment containing a targeting element was released from the genome using Sspl and Spel. ExoCET cloning was performed using 10 µg of restriction-digested genomic DNA and PCR-amplified p15A-cm vector. Targeting fragments and wild-type fragments cloned into the p15A vector can be separated by double streak and restriction enzyme digestion.

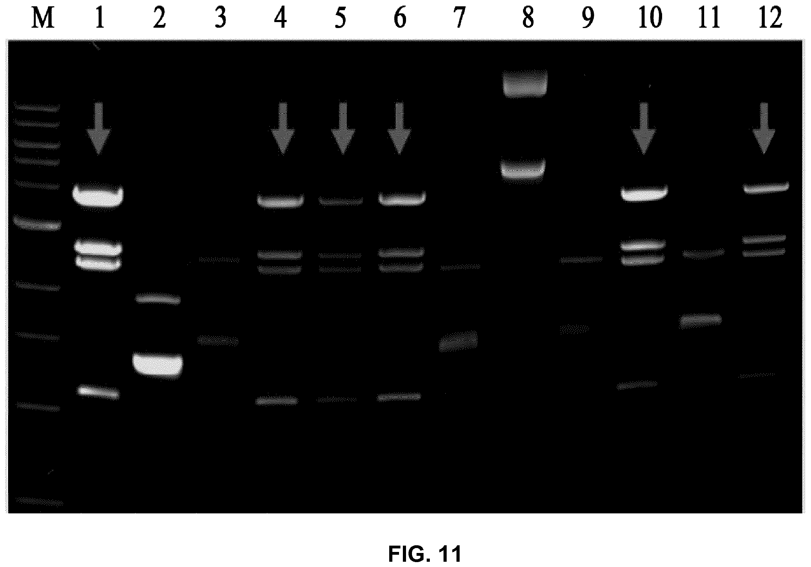

FIG. **11** shows EcoRV+PstI restriction analysis of chloramphenicol resistant colonies obtained by ExoCET genotyping of Klf4-Venus-neo-targeted mouse embryonic stem cells.

DNA recombineering is a genetic engineering technique for modifying DNA molecules in *E. coli* cells, which is mediated by homologous recombination of the phage syn/exo proteins (mainly Redα and Redβ)¹⁸⁻²². DNA recombineering was first discovered in the *E. coli sbcA* (recBC repressor) strain, which has an activity that efficiently mediates homologous recombination between DNA molecules with homology boxes²³. The *sbcA* strain was discovered in a classic experiment by AJ Clark looking for a homologous recombination pathway in *E. coli.* He used the recBC strain, which is very sensitive to DNA damage, to screen for its inhibitor, and found *sbcA* mutant strains with RecE and RecT expression activities ²⁴⁻²⁶. Subsequent studies have shown RecE and RecT are expressed by Rac phage integrated on chromosomes, which function identically to phage Redα and Redβ²⁷, and only 280 amino acids at the C-terminus of RecE protein are expressed in the sbcA mutant strain²⁸⁻³⁰. The truncated RecE is similar to Redα (266 amino acids) and is a 5' to 3' exonuclease³¹. RecT is similar to Redβ and is a single-strand annealing protein (SSAP)³². RecE/RecT and Redα/Redβ belong to the 5'to 3' exonuclease/SSAP syn/exo protein pair^{21, 33}, and a specific protein-protein interaction potential between each pair of proteins is necessary for homologous recombination of double-stranded DNA ^{29,34,35}. Redα/Redβ-mediated homologous recombination occurs mainly on the replication fork and requires simultaneous replication. Although the inventors initially discovered recombinant engineering techniques through truncated RecE/RecT, the inventors later used Redα/Redβ to modify DNA molecules because the latter were more efficient^{16, 38-42}. Nevertheless, the inventors are constantly studying the characteristics of RecE/RecT, and found that the 600 amino acid residues at the N-terminus of RecE changes their recombination activity from replication-dependent to replication-independent³. Therefore, two linear DNA molecules can form a circular plasmid by efficient homologous recombination through a very short homologous box. Compared with the Redα/Redβ recombineering, this linear-linear recombination mechanism has different applications, such as directly cloning large DNA fragments from genome^{3-6, 12} or performing multiple DNA fragment assembly ^{15,43}.

The disclosure provides a method of homologous recombination (linear-linear recombination) between two or more target linear nucleic acid molecules sharing at least one homologous sequence. The method comprises the mixture of target linear nucleic acid molecules treated with a first exonuclease; then the treated target linear nucleic acid molecules is subjected to homologous recombination in the presence of a second exonuclease and an annealing protein. The second exonuclease can be RecE, and the amino acid sequence of full length RecE from *E. coli* K12 is disclosed in WO2011/154927. Or the second exonuclease may also be truncated RecE, and the truncated forms of RecE including RecE protein consisting of amino acids 588-866, 595-866, 602-866 or 606-866²⁹.

Homologous recombination is mediated by the second exonuclease and the annealing protein. In some embodiments, the annealing protein used in the methods of the disclosure is the annealed protein disclosed in WO2011/154927. Particularly, the annealing protein is RecT or a fragment thereof (derived from Rac phage). More particularly, the annealing protein is the full length RecT and the second exonuclease is the full length RecE. However, any other suitable annealing protein can be used as long as the annealing protein interacts with the exonuclease used. Examples of other suitable annealing proteins are provided in WO 02/062988. Linear-linear recombination can occur in the host cells lacking RecT expression, such as *E*. *coli* strain GB2005, possibly due to the presence of certain endogenous RecT-like activities. However, the efficiency of linear-linear recombination mediated by full length RecE is significantly increased in the presence of RecT.

The methods of the disclosure can be affected in whole or in part in a host cell. Suitable host cells include cells of many species, including parasites, prokaryotes, and eukaryotes, but bacteria such as Gram-negative bacteria are preferred hosts. More particularly, the host cell is an enteric bacterial cell such as *Salmonella, Klebsiella, Bacillus, Neisseria, Photorhabdus* or *Escherichia Coli* cells (the method of the disclosure plays an effective role in all *E. coli* strains that have been tested). A preferred host cell is *E. coli* K12. However, it should be noted that the methods of the disclosure are equally applicable to eukaryotic cells or organisms, such as fungal, yeast, plant or animal cells. This system has been shown to be functional in mouse's ES cells and it is reasonable to speculate that it is also functional in other eukaryotic cells. The host cell is typically an isolated host cell, but can be unisolated host cells.

The host cell of the disclosure comprises a nucleic acid encoding an exonuclease (particularly full length RecE), an annealing protein (particularly RecT) and Redγ. In some embodiments, the host cell further comprises a nucleic acid encoding RecA. Particularly, the host cell expresses RecE, RecT and Redγ, and optionally RecA. More particularly, the host cell expresses RecE, RecT, Redγ and RecA.

The exonuclease, annealing protein, Redγ and/or RecA of the disclosure can be a recombinant expression product from a foreign DNA in a host cell, for example, expressed by a vector transformed into a host cell. An example of a suitable vector is the pSC101 plasmid, although other suitable vectors can also be used. Any suitable promoter can be used to drive the expression of these proteins. However, in the case of expressing RecE, an inducible promoter such as an arabinose-inducible promoter (P_{BAD}) or a rhamnose-inducible promoter (P_{RhaSR}) is preferred. These promoters are well known in the art.

The host cell of the disclosure expresses an exonuclease, an annealing protein, Redγ and/or RecA by the inducible promoters on a plasmid vector or a chromosome. Particularly, the exonuclease, annealing protein, Redγ and/or RecA are expressed in the host cell by a plasmid vector. More particularly, the exonuclease, annealing protein, Redγ and/or RecA are simultaneously expressed in the host cell by the plasmid vector and the chromosome.

The genome of the *E. coli* K12 host cell consists of an endogenous copy of the full-length recE gene and the recT gene, which are present in the Rac phage that has been integrated into the host genome. However, since the gene is silent, the expression of full-length RecE cannot naturally occur from the integrated gene. Thus, in embodiments where the 5' to 3'exonuclease is expressed by exogenous DNA, the method can be carried out in the absence of an endogenous recE gene.

Host cells transformed with the encoding as above nucleic acid molecule of the exonuclease are also provided. Particularly, the exonuclease is expressed by the nucleic acid molecule, and thus the disclosure also provides the host cell expressing the exonuclease enumerated in the method of the disclosure. The exonuclease is particularly expressed under the control of an inducible promoter, such as an arabinose-inducible promoter (P_{BAD}) or a rhamnose-inducible promoter (P_{RhaSR}).

In the foregoing embodiments, the methods of the disclosure may be affected in whole or in part in vitro. For example, purified 5' to 3' exonuclease and annealing protein (particularly purified RecE and RecT proteins) can be used, or an extract of *E. coli* cell expressing the 5' to 3' exonuclease and the annealing protein are used. When the method is carried out in vitro, it is advantageous to pretreat the first and second linear target nucleic acid molecules to expose single-stranded homologous ends.

Linear-linear recombination requires that at least one homologous sequence must be shared between the target linear nucleic acid molecules in which homologous recombination occurs. In some embodiments, the first target nucleic acid molecule shares a homologous sequence with the second target nucleic acid molecule to perform the linear-linear recombination between the first and second target nucleic acid molecules, to produce a linear product. In embodiments in which linear-linear recombination occurs between the first and second linear nucleic acids and one or more additional linear nucleic acids to form a linear product, each linear nucleic acid shares a homologous sequence with the linear nucleic acid that forms its neighbor in the linear products of the linear-linear recombination reaction. In embodiments in which linear recombination occurs between the first and second linear nucleic acids and one or more additional linear target nucleic acid molecules, to form a cyclic product, each linear nucleic acid shares a homologous sequence with linear nucleic acid that forms its neighbor in the cyclic product of the linear-linear recombination reaction. In some embodiments, the first target nucleic acid molecule and the second target nucleic acid molecule share two homologous sequences to perform a linear-linear recombination between the first and second target nucleic acid molecules, to form a cyclic molecule. Those skilled in the art know how to design homologous sequences to form linear or cyclic molecules.

Particularly, at least one homology box is at the very end of each linear fragment. When the homology boxes are at the very end of each linear fragment and the different homology boxes are at the other end, these homologous sequences or 'homologous boxes' produce the optimal configuration, and the construction of these homology boxes enables recombination to generate a ring. Linear recombination can occur when the homology box is not at the end, but the efficiency is reduced. Thus, in a preferred embodiment, at least one at least one homologous sequence is located at the outermost end of one or both ends of the target nucleic acid molecule. In some embodiments, the at least one homologous sequence is internal to the certain target nucleic acid molecule.

The homologous sequences of the disclosure are at least 4, at least 6, at least10, at least 20, at least 30, at least 40, at least 50, at least 60, at least 70, at least 80, at least 90, at least 100 nucleotides in length. For example, in some embodiments, the homologous sequences are 4-6, 6-9, 6-30, 6-100, 10-20, 20-29, 20-40, 20-50, 10-100, 25 -30, 25-40, 25-50, 30-40, 30-50, 40-50, 40-80 or more than 80 nucleotides. The efficiency of homologous recombination increases with the length of the homology boxes used, so longer homology boxes can be used.

'Homologous' between two nucleic acid molecules means that when the sequences of two nucleic acid molecules are aligned, there are many nucleotide residues that are identical at the same position in the sequence. The degree of homology is easy to calculate
(Computational Molecular Biology, Lesk, A.M.,ed., Oxford University Press, New York,1988; Biocomputing. Informati cs and Genome Projects, Smith,D.W.,ed., Academic Press,New York,1993;
Computer Analysis of Sequence Data,Part1,Griffin,A.M.,and Griffin,H.G.,eds., Humana Press,New Jersey,1994; Sequence Analysi s in Molecular Biology, von Heinje,G.,Academic Press,1987;and Seq uence Analysis Primer,Gribskov ,M.and Devereux,J.,eds.,M Stockton Press,New York,1991).

In some embodiments, the methods of the disclosure comprise joining together a plurality of linear nucleic acid molecules to form a circular nucleic acid molecule, such as a circular plasmid. Each target nucleic acid molecule shares a at least one homologous sequence with a target nucleic acid molecule that forms its neighbor in the resulting cyclic product and is subjected to linear-linear recombination in accordance with the methods of the disclosure. The number of target nucleic acid molecules is 2, 3, 4, 5, 6, 7, 8, 9, 10, 11, 12, 13, 14, 15, 16, 17, 18, 19, 20 or more.

In some embodiments, at least one of the target linear nucleic acid molecules comprises a selection marker to allow selection of the correct recombinant. Any suitable selection marker can be used in the disclosure. In some embodiments, the selection marker is an antibiotic resistance gene, e.g., a resistance gene for chloramphenicol, ampicillin, kanamycin, or blasticidin.

The target linear nucleic acid molecule can be derived from any suitable source. For example, nucleic acid sequences from eukaryotes or prokaryotes can be included. In some embodiments, the first target linear nucleic acid molecule is genomic DNA. Typically, the genomic DNA is a genomic DNA fragment. The genomic DNA particularly consists of a target sequence. In some embodiments, a genomic DNA fragment can be obtained by cleavage or digestion of genomic DNA (for example, using a restriction enzyme) to obtain a complete target sequence containing. In some embodiments, the first target linear nucleic acid molecule (such as, a genomic DNA fragment, a cDNA library member, or a BAC-derived fragment) comprises a target sequence of 2-kb or longer (e.g., 2.5-kb or longer, 4-kb or longer, 5-kb or longer, 7.5-kb or longer, 10-kb or longer, 15-kb or longer, 20-kb or longer, 25-kb or longer, 40-kb or longer, 50-kb or longer, 75-kb or longer or-100 kb or longer). Particularly, the target sequence is the entire region between the homology boxes at either end of the first target linear nucleic acid molecule. For example, a gene cluster encoding a secondary metabolite pathway or a fatty acid synthesis pathway. In some embodiments, the methods of the disclosure can be used to directly clone a DNA region from a human or non-human animal genome. For example, regenerative therapies for health research or for correction by gene targeting. For example, in some embodiments, the first target nucleic acid molecule comprises or consists of a genomic DNA fragment from a human or non-human animal. The genomic DNA fragment can comprise a target sequence, such as a gene comprising a mutation, where the mutation results in a disease or condition and the modification of the mutation to a wild type sequence can treat or prevent the disease or condition. In embodiments where the first target nucleic acid molecule is a genomic DNA fragment, the second target nucleic acid molecule is particularly a linear cloning vector.

In embodiments where the first target nucleic acid molecule is a genomic DNA fragment, The method comprises generating a first target nucleic acid molecule by digesting or cleaving genomic DNA to obtain a linear genomic DNA fragment comprising the target sequence, then, the first exonuclease is used to treat the mixture of the genomic DNA fragment and the linear cloning vector, processing the steps of cleaving the target nucleic acid molecule and annealing to ligate the target nucleic acid molecule, and then the mixture of the treated nucleic acid molecules is transferred into host cells. The second target nucleic acid molecule particularly comprises a selection marker.

In one embodiment, the methods of the disclosure comprise the step of ligation of DNA molecules in vitro.

The ligation process in vitro comprises exonuclease digestion followed by annealing.

The exonuclease is T4 polymerase.

The ligation process in vitro comprises Gibson assembly.

The ligation process in vitro comprises DNA synthesis by DNA polymerase with or without exonuclease followed by annealing.

The ligation process in vitro comprises annealing by a single-stranded annealing protein, such as RecA/RAD51, Redβ, RecT. Pluβ or RAD52.

Host cells used for homologous recombination are *E. coli* cells.

Host cells for homologous recombination are *E. coli cell* expressing full length RecE and/or RecT.

Host cells for homologous recombination are *E. coli cell* expressing full length RecE, RecT and/or Redγ.

Host cells for homologous recombination are *E. coli cell* expressing truncated RecE, RecT and/or Redγ.

Host cells for homologous recombination are any bacterial host cell expressing full length RecE and/or RecT.

Host cells for homologous recombination are *E. coli cell* expressing Redα, Redβ and/or Redγ.

The host cell for homologous recombination is *Saccharomyces cerevisiae* cells.

Kits for use in the disclosure are provided. In some embodiments, the kits comprise a nucleic acid encoding an exonuclease as described herein. In some embodiments, the kit comprises an exonuclease as described herein. Particularly, the first exonuclease is T4 DNA polymerase (T4pol), Klenow fragment of DNA Polymerase I (Kle), T7 DNA polymerase (T7pol), Exonuclease III (Exolll), Phusion DNA polymerase (Phu), T5 exonuclease (T5exo), T7 exonuclease (T7exo) and Lambda exonuclease (λexo), and the second exonuclease is full length RecE. More particularly, the kits comprise a host cell as described herein. For example, in some embodiments, the host cells in a kit comprises a nucleic acid encoding a full length RecE, RecT, Redy, and RecA described herein under the control of an inducible promoter. The kits may also include one or more pre-prepared linear cloning vectors.

Another preferred application of the disclosure relates to the assembly of linear nucleic acid molecules in synthetic biology, particularly linear DNA. Thus, in some embodiments, the first and second target nucleic acid molecules are linear, and the method further comprises contacting the first and second target nucleic acid molecules with one or more other linear target nucleic acid molecules (For example, 1, 2, 3, 4, 5, 6, 7, 8, 9, at least 10, at least 20 other target nucleic acid molecules) in the presence of a 5' to 3' exonuclease and an annealing protein, to produce a linear or circular product. In a preferred embodiment, homologous recombination between the first and second target nucleic acid molecules and one or more other target nucleic acid molecules results in the production of genes, operons, chromosomes or whole genomes. Synthetic biological assembly of DNA nucleic acids has been used to generate genes, operons, chromosomes, or recently used to generate whole genomes. In an embodiment of the disclosure, the combination of the first exonuclease and the second exonuclease significantly increases the assembly efficiency of the linear nucleic acid molecule, the disclosure will be a preferred method for the assembly of synthetic biological DNA in commerce and research.

Another preferred application of the disclosure is to construct a haplotype isogenic targeting vector, which can directly clone a 5 to 10-kb DNA fragment from mammalian genome using the method of the disclosure as an isogenic homology box, and these DNA fragments are not only the identical genes but also maintains a polymorphic haplotype, so the inventors call it a haplotype isogenic targeting vector, that is the so called haplotype isogenic targeting (HIT) vector. The selection marker and other functional elements are then inserted into the HIT vector by recombinant engineering to obtain a vector for targeting. Another preferred application of the disclosure is the genotyping of mammalian cells. The DNA fragment containing the complete targeting element is cloned from the genome of the possible target embryonic stem cells by the method of the disclosure, and the recombinant plasmid obtained by the cloning is subjected to restriction analysis and DNA sequencing, and the cell is successfully determined according to the result.

### Examples

### Materials and Method

### Strain and plasmid

*E. coli* GB2005 was derived from DH10B by deleting *fhuA, ybcC* and *recET* ^{3,16,44}. GB05-dir was derived from GB2005 by integrating the P_{BAD}-ETgA operon (full length *recE, recT, redγ* and *recA* under the arabinose-inducible P_{BAD} promoter)³ at the *ybcC* locus. GB08-red was derived from GB2005 by integrating the P_{BAD}-gbaA operon (*redγ, redβ, redα* and *recA* under the arabinose-inducible P_{BAD} promoter)¹⁶ at the *ybcC* locus. pSC101-BAD-ETgA-tet³ conveys tetracycline resistance and carries the P_{BAD}- full length ETgA operon and a temperature sensitive pSC101 replication origin which replicates at 30°C but not at 37°C so it can be easily eliminated from the host by temperature shift in the absence of selection.

### Preparation and digestion of genomic DNA

Gram-negative *Photobacterium phosphoreum* ANT-2200 and *Photorhabdus luminescens* DSM15139 were cultured overnight in 50 ml medium. After centrifugation the cells were resuspended thoroughly in 8 ml of 10 mM Tris-Cl (pH 8.0). Five hundred microliters of 20 mg ml⁻¹ proteinase K and 1 ml of 10% SDS were added and incubated at 50 °C for 2 h until the solution became clear. Genomic DNA was recovered from the lysate by phenol-chloroform-isoamyl alcohol (25:24:1, pH 8.0) extraction and ethanol precipitation. The DNA was dissolved in 10 mM Tris-CI (pH 8.0) and digested with BamHI+KpnI for cloning of the 14-kb *lux* gene cluster.

Gram-positive *Streptomyces albus* DSM41398 was cultured in 50 ml of tryptic soy broth at 30°C for 2 days. The genomic DNA was isolated according to the method described in ref. (10) with slight modification. After centrifugation the cells were resuspended thoroughly in 8 ml of SET buffer (75 mM NaCl, 25 mM EDTA, 20 mM Tris, pH 8.0) and 10 mg lysozyme was added. After incubation at 37°^{C} for 1 h, 500 µl of 20 mg ml⁻¹ proteinase K and 1 ml of 10% SDS were added and incubated at 50°C for 2 h until the solution became clear. Three and a half milliliters of 5 M NaCl was added into the lysate. Genomic DNA was recovered from the lysate by phenol-chloroform-isoamyl alcohol (25:24:1, pH 8.0) extraction and ethanol precipitation. The DNA was dissolved in 10 mM Tris-CI (pH 8.0).

Genomic DNA was purified from mouse melanoma B16 cells, human embryonic kidney 293T cells and human blood using Qiagen Blood & Cell Culture DNA Kits according to the manufacturer's instructions, except DNA was recovered from the Proteinase K treated lysate by phenol-chloroform-isoamyl alcohol (25:24:1, pH 8.0) extraction and ethanol precipitation. The DNA was dissolved in 10 mM Tris-CI (pH 8.0). Restriction digested genomic DNA was extracted with phenol-chloroform-isoamyl alcohol (25:24:1, pH 8.0) and precipitated with ethanol. The DNA was dissolved in 10 mM Tris-CI (pH 8.0). End cut pipette tips were used to avoid shearing genomic DNA.

The genomic DNA of *P. luminescens* DSM15139 was digested with Xbal for *plu3535-plu3532* cloning, and XbaI+XmaI for *plu2670* cloning. The genomic DNA of S. *albus* was digested with EcoRV or Cas9-gRNA complexes for cloning of the salinomycin gene cluster. The mouse genomic DNA was digested with Hpal for *Prkar1a* cloning, BamHI+KpnI for *Dpy30* cloning, and Swal for *Wnt4* or *Lmbr1I-Tuba1a* cloning. The human genomic DNA was digested with Spel for *DPY30* cloning, NdeI+BstZ17I for *IGFLR1-LIN37* cloning, BstZ17I for *IGFLR1-ARHGAP33* cloning and Ndel for *ZBTB32-LIN37* cloning. Digested genomic DNA was extracted with phenol-chloroform-isoamyl alcohol (25:24:1, pH 8.0) and precipitated with ethanol. The DNA was dissolved in ddH₂O and concentrated to 1 µg µl⁻¹. End cut pipette tips were used to avoid shearing genomic DNA. Ten micrograms of digested genomic DNA were used for ExoCET cloning.

### Cas9 digestion of S. albus genomic DNA

S. *pyogenes* Cas9 protein was purchased from New England Biolab. Cas9 digestion of S. albus genomic DNA was carried out in an 800 µL reaction system containing 80 µL of 10×Cas9 reaction buffer (NEB), 80 µg of genomic DNA, 40 µg of gRNA-2, 40 µg of gRNA-7 and 20 µg of Cas9. Since the cleavage efficiency of Cas9 was severely affected by the purity of the DNA substrate, in this experiment, the S. *albus* genomic DNA needed to be extracted three times with phenol-chloroform-isoamyl alcohol (25:24:1, pH 8.0) to ensure the cleavage efficiency of Cas9. After the resulting product was incubated at 37°C for 6 h, 100 µg of RNase A (Thermo Scientific) was added, and after incubation at 37°C for 1 h, 100 µg of proteinase K (Roche) was then added, and incubation was continued at 50°C for 1 h. The genomic DNA was then extracted once with phenol-chloroform-isoamyl alcohol (25:24:1, pH 8.0), and after ethanol precipitation, it was dissolved in an appropriate amount of ddH₂O to a final concentration of about 1 µg uL⁻¹. Finally, 10 µg of genomic DNA cleaved by Cas9 protein was used for the cloning experiment of the method of the disclosure.

### Preparation of linear cloning vector

Using p15A-Pamp-luxABECD plasmid (Genebridge) as a template, the p15A-cm vector was PCR-amplified with PrimeSTAR Max DNA Polymerase (Takara), the primers used (Table 1) consist of 80 nucleotide homology boxes and were purified by PAGE. The PCR product eliminated the interference of the primers on subsequent experiments by gel recovery. The kit used was QIAquick gel extraction kit (Qiagen). Finally, the DNA was eluted with ddH₂O at a concentration of approximately 200 ng/µL, and 200 ng was used for the ExoCET cloning experiment.

The pBeloBAC11 vector used to clone the salinomycin gene cluster and the pBAC2015 vector used to clone plu3535-3532 were constructed according to the references^{43,46}. BAC vectors were linearized with BamHI to expose both homology arms, and extracted with phenol-chloroform-isoamyl alcohol (25:24:1, pH 8.0) and precipitated with isopropanol. The DNA was dissolved in ddH2O and concentrated to 1 µg/µL. One microgram of linear BAC vectors were used for ExoCET cloning.

**Table 1 Oligonucleotides amplified by p15A-cm linear vector**

| Gene | Primer | Sequences (5'-3') |
|---|---|---|
| *plu2670* | plu2670-1 | ctgggaaaactacggtgagcatatgtttaccacttcttcagagaaccgtgaatattgcg ttaaaccgatgaactgcccgggTTACGCCCCGCCCTGCCACTC (SEQ. ID. NO. 1) |
| | plu2670-2 | aaatccatgattaccaacccactcccaaagatattataaaatctcaacatgctgatttg atcctttggaatgggctaaatctagaGGTAACGAATCAGACAATTGAC G (SEQ. ID. NO. 2) |
| Mouse *Prkar1a* | mPrkar1a-1 | agatgacgccctctccactctgcataccaattccacataaccactcttcaaattataat gctcacagaccctctaaggttTTACGCCCCGCCCTGCCACTC (SEQ. ID. NO. 3) |
| | mPrkar1a-2 | catagtgacccattgttatagagtacttagctatgcctcagagtaatgctcttgatctagg tgctttctttttacttgttGGTAACGAATCAGACAATTGACG (SEQ. ID. NO. 4) |
| Mouse *Dpy30* | mDpy30-1 | ggattttataagtgaagggtgacatttgtagtattccttaccccgtctggttcctcatacgc attaactcataatccttggtaccTTACGCCCCGCCCTGCCACTC (SEQ. ID. NO. 5) |
| | mDpy30-2 | tgccctttcagaagacctaggtttgattcctagcacccactgatgctaacaaccagac ataatgccagttctaggggatccGGTAACGAATCAGACAATTGACG (SEQ. ID. NO. 6) |
| Mouse *Wnt4* | mWnt4-1 | gtttaactcccttaacacacacacacacacatgcacacgcacatgcacacgcgcac tcacaccacaaaatcacacaatttTTACGCCCCGCCCTGCCACTC (SEQ. ID. NO. 7) |
| | mWnt4-2 | ctgtgctcagagctttgagtgccctacatataacaggacacaaactcttccttactttctg attgccacatggtccatttGGTAACGAATCAGACAATTGACG (SEQ. ID. NO. 8) |
| *Mouse Lmbr1I-Tuba1a* | mLT-1 | ctatgccataggaatttggaggaaattaggcttactggtccattaattatttccaacgga tgcagtcagaatgtatatttTTACGCCCCGCCCTGCCACTC (SEQ. ID. NO. 9) |
| | mLT-2 | ttagagcagacagtgctcttaaccgttgagccatctctccagtccctggacacactgat tttaagttgacattaaaatttGGTAACGAATCAGACAATTGACG (SEQ. ID. NO. 10) |
| human *DPY30* | hDpy30-1 | ccaccacgccaggctaattttagcttaataccctattacctctcaggtgtgtctaaattttt ctttgatggaaaaactagtTTACGCCCCGCCCTGCCACTC (SEQ. ID. NO. 11) |
| | hDpy30-2 | agttatgtcagtttgctattataatgtttattattattccttgaataaaaaagaatatgcacct aataaacgttgactagtGGTAACGAATCAGACAATTGACG (SEQ. ID. NO. 12) |
| human *IGFLR1 -LIN37* | hIL-1 | Agtggccctagagggtcggtaaggagccaagggcctatgcaaggatgctcacac acctcccatccccaccttcccagacacatatgTTACGCCCCGCCCTGCC ACTC (SEQ. ID. NO. 13) |
| | hIL-2 | gtgctgtccaagcttggctcatctggggtttgctgggcttaacacccaataaagaacttt gctgactactaagcccagtaGGTAACGAATCAGACAATTGACG (SEQ. ID. NO. 14) |
| human *IGFLR1 ARHGA P33* | hIA-1 | acggccggcgactgctcctcagtgaggaggcgtcactcaatatccctgcagtggcg gccgcccatgtgatcaaacggtaTTACGCCCCGCCCTGCCACTC (SEQ. ID. NO. 15) |
| | hIA-2 | gtgctgtccaagcttggctcatctggggtttgctgggcttaacacccaataaagaacttt gctgactactaagcccagtaGGTAACGAATCAGACAATTGACG (SEQ. ID. NO. 16) |
| human *ZBTB32 -LIN37* | hZL-1 | cctagagggtcggtaaggagccaagggcctatgcaaggatgctcacacacctccc atccccaccttcccagacacatatgTTACGCCCCGCCCTGCCACTC (SEQ. ID. NO. 17) |
| | hZL-2 | aagaactagactagaagttggaaaacaggtttcactgcatcccttcacagtgaatgg cccttctcttcagcactcatatgGGTAACGAATCAGACAATTGACG (SEQ. ID. NO. 18) |

Lower-case letters in Table 1 represent the homologous box sequence.

### Preparation of PCR products for multiple fragment assembly

The DNA fragments with 40bp homologies used in the multi-piece assembly experiment were PCR amplified using genomic DNA of P. luminescens DSM15139 as the template and PrimeSTAR Max DNA Polymerase (Takara) according to the manufacturer's instructions. The PCR products were extracted from agarose gels after electrophoresis and purified using the QIAquick gel extraction kit (Qiagen) according to the manufacturer's instructions, except that DNA was eluted from the column with ddH₂O and concentrated to 200 ng µL⁻¹. 250 ng of each fragment was used for DNA assembly.

### Preparation of mVenus-PGK-neo DNA cassette

The mVenus-PGK-neo cassette was amplified from pR6K-2Ty1-2PreS-mVenus-Biotin-PGK-em7-neo¹⁷ with PCR using the proof reading PrimeSTAR Max DNA Polymerase (Takara) according to the manufacturer's instructions. The primers are listed in Table **1.** The PCR products were purified with QIAquick PCR Purification Kit (Qiagen) according to the manufacturer's instructions, except that DNA was eluted from the column with ddH2O and concentrated to 100 ng µL⁻¹. Two hundred nanograms of the cassette was used for recombineering.

### In vitro assembly

Ten micrograms of genomic DNA and 200 ng of 2.2-kb p15A-cm linear vector (1 µg of 8-kb linear BAC vector) were assembled in 20 µL reactions consisting of 2 µL of 10 × NEBuffer 2.1 and 0.13 µL of 3 U µL ⁻¹ T4pol (NEB, cat. no. M0203). Assembly reactions were prepared in 0.2 ml PCR tubes and cycled in a thermocycler as follows: 25 °C for 1 h, 75 °C for 20 min, 50 °C for 30 min, then held at 4°C. For multi-piece assembly, 250 ng of each fragment was added and a chew-back time of 20 min was used. The in vitro assembly products were desalted at room temperature for 30 min by drop dialysis against ddH₂O using Millipore Membrane Filters (Merck-Millipore, cat. no. VSWP01300) prior to electroporation. All experiments were performed in triplicates.

Assembly reactions with other exonucleases were cycled as follows: T5exo: 50°C for 30 min, then held at 4°C; T7exo: 25°C for 20 min, 50°C for 30 min, then held at 4°C; Kle, T7pol and λexo: 25 °C for 20 min, 75°C for 20 min, 50 °C for 30 min, then held at 4°C; Exolll: 37 °C for 20 min, 75°C for 20 min, 50 °C for 30 min, then held at 4 °C; Phu: 37 °C for 20 min, 50°C for 30 min, then held at 4°C. Gibson assembly was performed at 50 °C for 30 min with Gibson Assembly Master Mix (NEB, cat. E2611), then held at 4°C.

### Preparation of electrocompetent E. coli cells

*E. coli* GB05-dir containing plasmid pSC101-BAD-ETgA-tet was cultured (OD600=3-4) overnight at 30°C in LB supplemented with 4 µg/mL tetracycline. 40 µL of overnight culture (OD600=3-4) was transferred to 1.4 mL LB supplemented with appropriate antibiotics, then the mixture was placed on an Eppendorf thermomixer at 30°C and incubated at 950 rpm for 2 h (OD600 =0.35-0.4). 35 µL of 10% L-arabinose (w/v, in ddH₂O) was added to induce expression of recombinant enzyme (ETgA or gbaA), and the incubation was continued for 40 min at 37°C (OD600= 0.7 to 0.8). The cells were collected by centrifugation at 9,400 g for 30 sec at 2°C.

The supernatant was discarded and the cell pellet was suspended in 1 mL of ice-cold ddH₂O. The cells were collected by centrifugation at 9,400 g for 30 sec at 2°C. The supernatant was discarded and the cell pellet was suspended in 1 mL of ice-cold ddH₂O. The cells were repeatedly centrifuged, resuspended, and centrifuged again, and the cells were suspended with 20 µL of ice-cold ddH₂O. Then 5 µL of desalted in vitro assembled product was added, while in the mVenus-PGK-neo element insertion experiment, the mixture of 200 ng of plasmid and 200 ng of PCR product was added. The mixture of cells and DNA was transferred to a 1-mm cuvettes and electroporated with an Eppendorf electroporator 2510 at a voltage of 1350 V, a capacitance of 10 uF, and a resistance of 600 Ω. 1 mL LB was added to the electric shock cup, washed the cells and transferred it to a 1.5 mL tube with holes, and then placed on the Eppendorf thermomixer at 950 rpm for 1 h at 37°C. Finally, an appropriate amount of the bacterial solution was spread to an LB plate supplemented with a suitable antibiotic (15 µg/mL chloramphenicol or 15 µg/mL kanamycin) and incubated at 37°C overnight.

### Example 1

Concerted action of in vitro assembly and full length RecE/RecT improves the efficiency of direct cloning.

Inventors tested a series of exonucleases and annealing methods by direct cloning of a 14-kb *lux* gene cluster of *Photobacterium phosphoreum ANT-2200*⁴⁷ (FIG. 1A). In this cloning experiment, 10 µg of ANT-2200 genomic DNA digested using BamHI and Kpnl is mixed with 200 ng of 2.2-kb p15A-cm linear vector. The linear vector has a sequence at both ends that is identical to the ends (homologous box) of the genomic fragment containing the *lux* gene cluster produced by restriction digestion. The linear vector is homologously recombined with the target DNA fragment through the homologous boxes at both ends, to form a final circular plasmid. The cloning vector and BamHI+KpnI digested genomic DNA were first treated in vitro with different exonuclease enzymes, and then the reaction product after in vitro treatment was transformed into *E. coli* expressing the RecE/RecT recombinase. The tested exonucleases include: T4 DNA Polymerase (T4pol), Klenow Fragment of DNA Polymerase I (Kle), T7 DNA Polymerase (T7pol), Exonuclease III (Exolll); Phusion DNA polymerase (Phusion DNA polymerase; Phu), T5 Exonuclease (T5exo); T7 Exonuclease (T7exo) and lambda exonuclease (λexo) (FIG. 2A). The results showed that T4pol, Kle, T5exo and T7exo significantly improves the efficiency of direct cloning (FIG. 2A-E). The annealing rate after exonuclease digestion have no effect on cloning efficiency, so the inventors choose a default cooling rate (2°C s⁻¹) in the Eppendorf MC nexus PCR machine (FIG. 3). The effect of the concentration, reaction temperature and reaction time of the T4pol on direct cloning efficiency were tested (FIG. 1C, d and FIG. 2F). The efficiency of the direct cloning using the full-length RecE/RecT alone, T4pol in vitro annealing alone, and combining T4pol in vitro annealing with full length RecE/RecT were compared (FIG. 1F). When RecE/RecT is used alone, the 14-kb *lux gene* cluster is directly cloned from the chromosome of the luminescent bacterium to the p15A vector with a high accuracy (427), but the direct cloning assembling the reaction products after T4pol in vitro assembly into a standard engineering *E. coli,* is much more efficient (427 vs 4,880). The results showed that: (1) *E. coli* endogenous DNA repair system can skillfully close the plasmid backbone; (2) T4pol is highly efficient in in vitro assembly (4880); (3) ExoCET technique that combines T4pol in vitro assembly with RecE/RecT intracellular recombination is more efficient than either technique alone (32500).

### Example 2

### Effect of homology boxes on cloning efficiency

The longer the homology box, the higher the cloning efficiency (FIG. 1 B). The inventors placed the 80 bp homology box at one end of the cloning vector at a position of 1-kb inside the gene cluster, or both ends at a position of 1-kb inside the gene cluster, and then compared the efficiency of ExoCET with the efficiency of T4pol and RecET (FIG. 5b). When both homologous boxes of the vector were at the very end, the inventors obtained the same results as before. However, when one or two homology boxes were placed inside 1-kb of the gene cluster, the cloning efficiency of treatment with T4pol alone was very low, indicating that the annealing after T4pol exonuclease treatment depended on complementary pairing of the terminal DNA sequence. It was worth noting that the efficiency of RecET recombination had little to do with the position of the homology box. The experimental results obtained when one homology box was at the end and the other homology box was inside were thought-provoking. When there was only one terminal homology box between the vector and the genomic DNA fragment, the efficiency of ExoCET was 12 times than RecET, which indicated that T4pol in vitro treatment enabled two DNA molecules to be efficiently ligated through annealing one end before the electroporation and the RecET recombination. The above data indicated that T4pol could only act on the terminal homology box, and the recombination of the internal homology box required the action of RecET. Therefore, the inventors had concluded that the main contribution of T4pol to ExoCET was to significantly increase their co-transformation efficiency by annealing one end of two linear DNA molecules. RecET could then be used to facilitate reorganization at the other end. In the cloning experiment of the 14-kb *lux gene* cluster, when both homology boxes were located at the very end of the target digested genomic fragment, the cloning efficiency of ExoCET was 6-8 times than T4pol (FIG. 1F and 5B), which indicated that most of the in vitro assembled products had only one end joint together (>85%), and the recombinant plasmid produced by RecET after co-transformation of the in vitro assembled cyclic product and two linear DNA molecules contributed little to the high efficiency of ExoCET. When designing an ExoCET direct cloning experiment, the position of the homology box position was very important for cloning efficiency. To achieve the highest cloning efficiency, both homology boxes were placed at the very end of the target DNA fragment. In fact, in order to exert the effect of T4pol exonuclease and annealing in vitro, at least one homology box was placed at the end. However, another homology box could be placed inside the target DNA fragment because RecE/RecT could localize it and recombine it. This was very advantageous for the use of direct cloning to construct expression vectors, since one of the homology boxes could be placed at the very end of the 3' end of the target fragment, and the 5' end of the target gene could be placed directly under the control of the promoter and ribosome binding sites using internal homology boxes.

### Example 3

### RecE and RecT were both required for ExoCET

Since RecT is a single-stranded DNA annealing protein, the inventors hypothesized that RecT may anneal the single-stranded DNA region produced by T4pol (3' exonuclease), so RecE may not be required in the ExoCET technique system. In order to verify this conjecture, the inventors transformed the T4pol-treated DNA substrate into *E. coli* cell expressing RecT and Redγ (pSC101-Tg) and not expressing RecE, and found no interaction between RecT and T4pol. Therefore, both RecE and RecT were required for ExoCET (FIG. 5C). RecA had a certain improvement in the efficiency of direct cloning.

### Example 4

### Verification of direct cloning of large DNA fragments

In order to verify the superiority of ExoCET technique, the inventors used it to do some experiments that were previously difficult to do with RecET technique. There are two large gene clusters on the genome of *Photorhabdus luminescens:* 37.5-kb *plu3535-3532* and 52.6-kb *plu2670.* It was very difficult for the inventors to directly clone these two gene clusters with RecET technique, and the efficiencies were only 2/12 and 0/48³, respectively. While the inventors using ExoCET technique achieved correct rates of 10/12 and 11/17, respectively (Table 2).

**Table 2 Large fragments of genomic DNA cloned directly from bacteria, mammalian cells, and human blood using ExoCET**

| Target gene | Source | Genome (Mb) | Genomic restriction enzyme | Size (kb) | Vector | Number of colonies (/mL) | Correct number/Detection number |
|---|---|---|---|---|---|---|---|
| *plu3535-3532* | *(Photorhabdus luminescens)* DSM15139 | 5.69 | XbaI | 38 | pBAC2 015 | 1815±132 | 10/12 |
| *plu2670* | *(Photorhabdus luminescens)* DSM15139 | 5.69 | XbaI+X mal | 53 | p15A | 1152±211 | 11/17 |
| Salinomycin gene cluster | (*Streptomyces albus*) DSM41398 | 8.38 | EcoRV | 106 | pBeloB AC11 | 425±91 | 2/24 |
| Salinomycin gene cluster | (*Streptomyces albus*) DSM41398 | 8.38 | Cas9 | 106 | pBeloB AC11 | 260±14 | 1/24 |
| *Prkar1a* | Mouse melanoma B16 cells | 2800.06 | Hpal | 8 | p15A | 205±17 | 10/12 |
| *Dpy30* | Mouse melanoma B16 cells | 2800.06 | BamHI+ Kpnl | 8.7 | p15A | 273±18 | 9/12 |
| *Wnt4* | Mouse melanoma B16 cells | 2800.06 | SwaI | 45 | p15A | 76±16 | 8/25 |
| *Lmbr1I-Tuba1a* | Mouse melanoma B16 cells | 2800.06 | Swal | 53 | p15A | 52±6 | 1/12 |
| *DPY30* | Human embryonic kidney 293T cells | 3221.49 | Spel | 9.1 | p15A | 40±10 | 17/24 |
| *DPY30* | Human blood | 3221.49 | Spel | 9.1 | p15A | 45±2 | 5/24 |
| *IGFLR1-LIN37* | Human blood | 3221.49 | Ndel+BstZ17I | 14 | p15A | 320±67 | 9/48 |
| *IGFLR1-ARHGAP3 3* | Human blood | 3221.49 | BstZ17I | 41 | p15A | 275±76 | 5/48 |
| *ZBTB32-LIN37* | Human blood | 3221.49 | Ndel | 45 | p15A | 115±35 | 2/48 |
| *Oct4-Venus* | Mouse R1 embryonic stem cell | 2800.06 | EcoRV + Pacl | 9.6 | p15A | 34±1 | 9/36 |
| *Nanog-Cherry* | Mouse R1 embryonic stem cell | 2800.06 | Ndel | 13 | p15A | 49±12 | 17/54 |
| *Gata2-Venus* | Mouse R1 embryonic stem cell | 2800.06 | BstZ17I | 16.8 | p15A | 212±27 | 5/45 |
| *MII4(1)* | Mouse R1 embryonic stem cell | 2800.06 | Sspl+Spel | 17.1 | p15A | 127±38 | 7+3/24 |
| *MII4(2)* | Mouse R1 embryonic stem cell | | | | | 323±65 | 2+2/36 |
| *MII4(3)* | Mouse R1 embryonic stem cell | | | | | 142±27 | 6+9/72 |
| *MII4(4)* | Mouse R1 embryonic stem cell | | | | | 483±91 | 3+5/36 |

Furthermore, previously the inventor failed the attempt which directly clone the 106-kb *salinomycin* gene cluster from *Streptomyces albicans* genome. So, the inventor had to divide it into three fragments and then performed cloning step by step, and then integrated it into a complete gene cluster⁴³. However, through ExoCET, the inventors could directly clone the 106-kb *salinomycin* gene cluster into the BAC vector by using a BAC vector with a homology box and EcoRV digested genomic DNA, and obtained the correct rate of 2/24 (Table 2 and FIG. 6). Since there was an EcoRV restriction site on each side of the 106-kb *salinomycin* gene cluster, the inventors could release it from the chromosome and clone it into the vector. When cloning large DNA fragments, it was sometimes difficult to find suitable restriction sites on both sides of the target DNA fragment, but the use of programmable nucleases could eliminate the restriction on restriction sites, especially is an RNA-mediated endonuclease-Cas9^{48,49}. In order to test this idea, the inventors used Cas9 to release the 106-kb *salinomycin* gene cluster from the chromosome where very closed to the EcoRV, and then cloned the same 106-kb DNA fragment into the BAC vector using the same BAC vector. A similar cloning efficiency was finally obtained (Table 2 and FIG. 6). Therefore, ExoCET had a significantly superior performance in direct cloning of large DNA fragments compared to RecET.

Next, the inventors tested whether the efficiency of ExoCET could meet the requirement of directly cloning large DNA fragments from mammalian genome. The inventors used Swal to release a 45-kb fragment containing the *Wnt4* gene from the mouse genome (FIG. 7A), and the inventor obtained the correct rate of 8/25 through ExoCET (FIG. 7B). The inventors also tested the use of Gibson assembly¹ to clone this DNA fragment. Gibson assembly uses T5exo, Phusion DNA polymerase and Taq DNA ligase to assemble DNA molecules with homology boxes between each other. The inventors obtained a large number of colonies (181,000 and 257,000) by transforming the Gibson assembled DNA product into arabinose-induced and non-induced *E. coli* GB05-dir containing pSC101-BAD-ETgA-tet. And 60 colonies were detected and no correct clone was obtained (FIG. 7B), and all were p15A empty vectors of self-circularization.

### Example 5

### Assembly of DNA fragments using ExoCET

Gibson was a method for multiple fragment DNA assembly, so the inventors compared ExoCET and Gibson through some DNA multiple fragment assembly experiments (FIG. 7C). These DNA fragments were amplified by PCR and had a 40-bp homology box at the end. The efficiency of ExoCET and Gibson assembly was good in the 7-fragment and 10-fragment assembly experiments. When Gibson's in vitro assembled products were transformed into *E. coli* cell expressing RecE, RecT, Redγ and RecA (Gibson and ETgA), assembly efficiency and accuracy were significantly improved (FIG. 7D). ExoCET couldn't assemble more than 13 DNA fragments, Gibson couldn't assemble more than 16 DNA fragments, while Gibson and ETgA could assemble at least 20 DNA fragments into a 54.9-kb plasmid. Therefore, combining in vitro assembly with RecET recombination, the advantages for DNA assembly were obvious.

### Example 6

### Construction of haplotype isogenic targeting vector using ExoCET

ExoCET could also be used to directly clone DNA fragments from mammalian genomes including blood, disease-associated cell lines, etc. to facilitate haplotype studies of SNPs and to rapidly construct haplotype syngeneic (HIT) targeting vectors for targeting of nuclease-mediated human stem cells. The importance of human stem cells isolated from patients, cord blood or somatic cell reprogramming in biomedical research had received more and more attention. The research on the precise modification of stem cell genome had also received widespread attention. Transforming the human genome was more challenging than structuring the genome of experimental mice because human genetic diversity was complex. The importance of isogenicity (sequence similarity) for homologous recombination was realized many years ago when people use mouse embryonic stem cells for gene targeting¹⁴.

The effect of sequence mismatched homologous recombination had not been well studied. For example, how much recombination efficiency could a single mismatch (a SNP or an indel) reduce? How did the distance of the mismatched recombination site affect the recombination efficiency? How did multiple mismatches affect recombination efficiency? These issues had not yet been clarified. In any case, the use of identical sequences in gene targeting was clearly highly recommended, so ExoCET was an effective way to quickly obtain ideal homology boxes. Unlike the method of amplifying homology boxes from the genome by PCR, ExoCET was not limited by fragment size, did not introduce mutations, and was capable of maintaining a DNA haplotype. Furthermore, the ends of the homology boxes could also be selected according to the manner of genotyping (such as Southern blotting or ligation PCR), so the length of the homology boxes could be optimized. ExoCET therefore offers advantages for individualized genomic surgery, especially when combined with CRISPR/Cas9¹⁵.

The inventors used ExoCET to construct isogenic targeting vectors to engineer mammalian genomes. Given the experience⁵⁰ in mouse embryonic stem cell research, the inventors aimed to clone a 5 to 10-kb DNA fragment directly from human or mouse genome as an isogenic homology box (FIG. 8A and FIG. 9A). It is noteworthy that these DNA fragments were not only the same gene but also maintain the polymorphic haplotype, so the inventors called it the HIT (haplotype isogenic targeting) vector. The inventors directly cloned 8-9-kb DNA fragments from the human genomes (in vitro cultured cell lines and human blood) and mouse (in vitro cultured cell lines) by ExoCET (FIG. 8B and FIG. 9B). Selection markers and other functional elements¹⁶ were then inserted into the HIT vector by Redαβ recombineering (FIG. 8C and FIG. 9C).

### Example 7

### Genotyping Mammalian Cells Using ExoCET

ExoCET could also be used as the most reliable method for genotyping a modified genome, while Southern blotting and ligation PCR could produce false positive signals. Since long range PCR was prone to false positive signals in mammalian genotyping studies, the inventors previously wanted to confirm the Kmt2d-AID-neo-targeted mouse embryonic stem cells screened by long-segment PCR by Southern blotting. However, the inventors never got a good probe. Therefore, the inventors cloned a DNA fragment containing the entire targeting element from the genome of four possible Kmt2d-AID-neo-targeted mouse embryonic stem cells using the ExoCET method shown in FIG. 10A. The recombinant plasmid cloned by ExoCET was subjected to restriction analysis and DNA sequencing, and the results showed that the four cells were successfully targeted and were single-targeted (FIG 10B). In addition, the inventors have successfully used ExoCET to re-validate Oct4-Venus-neo, Nanog-Cherry-neo, Gata2-Venus-neo and Set1b-TC-neo target mouse embryonic stem cells which obtained previously (Table 3). These target cells previously verified by Southern blotting. These results indicated that there was no site restriction on ExoCET genotyping. The inventors previously failed to determine whether a Klf4-Venus-neo-targeted mouse embryonic stem cell was successfully targeted, because long-range PCR and Southern blotting had not obtained an exact signal. The inventors did not clone a DNA fragment having kanamycin resistance in the corresponding region on the genome by using ExoCET (Table 3). Restriction analysis of the cloned chloramphenicol-resistant plasmid found that 50% of them contained the wild-type DNA sequence (FIG. 11). Therefore, the inventors known exactly that this cell was not correctly targeted.

**Table 3 Experimental data of ExoCET genotyping**

| Mouse embryonic stem cell (Amount of transformed DNA) | Genomic restriction enzyme | Size (kb) | Number of colonies on chloramphenicol plates (/mL) | Kanamycin and chloramphenicol plates/Double streak (Km/Cm) | Correct rate of colonies on the double-antiplate of kanamycin + chloramphenicol* |
|---|---|---|---|---|---|
| Oct4-Venus-neo #7 (R1) (10 µg genomic DNA+ 250 ng vector) | EcoRV+P acl | 9.6 | 34 ± 1 | 9/36 | 9/9 |
| Nanog-Cherry-neo #18 (R1) (7.7 µg genomic DNA+ 400 ng vector) | Ndel | 13 | 49 ± 12 | 17/54 | 7/17 ** |
| Gata2-Venus-neo #19 (GM8) (1.5 µg genomic DNA+ 500 ng vector) | BstZ17I | 16.8 | 212 ± 27 | 5/45 | 5/5 |
| Set1 b-TC-neo #4 (R1) (10 µg genomic DNA+ 250 ng vector) | Asel | 24 | 49 ± 8 | 1/36 | 1/1 |
| Klf4-Venus-neo #9 (R1) (10 µg genomic DNA+ 250 ng vector) | Aflll | 10.2 | 18 ± 4 | 0/36 | - |

| | | | | | |
|---|---|---|---|---|---|
| * in Table 3 means after restriction analysis. ** in Table 3 means the remaining 10 were intramolecular recombination (containing 11 direct repeats greater than 40 bp in the cloned target sequence). | | | | | |

**Table 4 The amount of Oct4-Venus-neo targeted mouse embryonic stem cell genomic DNA required for optimizing ExoCET genotyping**

| 500 ng of genomic DNA digested with EcoRV and Pacl was mixed with the amount of p15A-cm vector shown below | | |
|---|---|---|
| Vector | Number of colonies on kanamycin and chloramphenicol double-resistance plate (/mL) | Correct number/detection number* |
| 500 ng | 12 | 3/3, 100% |
| 1000 ng | 10 | 3/3, 100% |
| 2000 ng | 8 | 3/3, 100% |

| 1000 ng of genomic DNA digested with EcoRV and Pacl was mixed with the amount of p15A-cm vector shown below | | |
|---|---|---|
| Vector | Number of colonies on kanamycin and chloramphenicol double-resistance plate (/mL) | Correct number/detection number* |
| 500 ng | 10 | 3/3, 100% |
| 1000 ng | 10 | 3/3, 100% |
| 2000 ng | 10 | 3/3, 100% |

| | | |
|---|---|---|
| * in Table 4 means after restriction analysis. | | |

ExoCET genotyping did not produce a false positive signal compared to long range PCR. Compared to Southern blotting, ExoCET genotyping is simpler and did not require cumbersome screening of hybridization probes. In ExoCET genotyping, restriction enzyme sites for the release of intact targeting elements were easily available, and in the case of well-prepared genomes, genotyping results were obtained in three days. More importantly, ExoCET never produced a false positive signal. Since the targeting element had a selection marker, as long as 500 ng of restriction enzyme genomic DNA was sufficient to obtain better cloning efficiency (Table 4). To increase the throughput of ExoCET genotyping, cells cultured in 96-well plates can be used.

### Example 8

### ExoCET cloning technique applied to metagenomic samples

Functional analysis of whole genome sequencing results requires a simple and rapid method of expression vector construction. According to the method of the disclosure, the inventors can clone a DNA fragment of up to 50-kb from a 3.0×10⁹-bp genome. To this end, the inventors diluted 1 ng of *P. phosphoreum* genomic DNA into 10 µg of *Bacillus subtilis* genomic DNA to mimic the metagenomics. The experiment successfully cloned the 14-kb *lux* gene cluster by ExoCET and obtained considerable efficiency (Table 5). Environmental samples usually contained more than 10⁴ species⁵¹⁻⁵³, so the results can motivate the inventors to apply ExoCET cloning technique to metagenomic samples.

**Table 5 The 14 kb lux gene cluster was cloned directly from the diluted P. phosphoreum genome using ExoCET**

| *P. phosphoreum* (ng) (BamHI+KpnI) | *B. subtilis* (µg) (BamHI) | Vectors | Number of colonies (/mL) | Correct number/Detection number |
|---|---|---|---|---|
| 10 | 10 | p15A-cm | 200±2 | 7/12 |
| 5 | 10 | p15A-cm | 142±22 | 5/12 |
| 2 | 10 | p15A-cm | 102±8 | 2/12 |
| 1 | 10 | p15A-cm | 104±18 | 2/24 |

### References:

1. Gibson, D.G. et al. Enzymatic assembly of DNA molecules up to several hundred kilobases. Nat. Methods 6, 343-345 (2009).
2. Shao, Z. & Zhao, H. DNA assembler, an in vivo genetic method for rapid construction of biochemical pathways. Nucleic Acids Res. 37, e16 (2009).
3. Fu, J. et al. Full-length RecE enhances linear-linear homologous recombination and facilitates direct cloning for bioprospecting. Nat Biotechnol 30, 440-446 (2012).
4. Tang, Y. et al. Heterologous expression of an orphan NRPS gene cluster from Paenibacillus larvae in Escherichia coli revealed production of sevadicin. J. Biotechnol. 194, 112-114 (2015).
5. Bian, X. et al. Direct cloning, genetic engineering, and heterologous expression of the syringolin biosynthetic gene cluster in E. coli through Red/ET recombineering. ChemBioChem 13, 1946-1952 (2012).
6. Bian, X. et al. Heterologous production of glidobactins/luminmycins in Escherichia coli Nissle containing the glidobactin biosynthetic gene cluster from Burkholderia DSM7029. Chembiochem 15, 2221-2224 (2014).
7. Bian, X. et al. In vivo evidence for a prodrug activation mechanism during colibactin maturation. Chembiochem 14, 1194-1197 (2013).
8. Larionov, V. et al. Specific cloning of human DNA as yeast artificial chromosomes by transformation-associated recombination. Proc. Natl. Acad. Sci. USA 93, 491-496 (1996).
9. Lee, N.C., Larionov, V. & Kouprina, N. Highly efficient CRISPR/Cas9-mediated TAR cloning of genes and chromosomal loci from complex genomes in yeast. Nucleic Acids Res. 43, e55 (2015).
10. Kouprina, N. & Larionov, V. Transformation-associated recombination (TAR) cloning for genomics studies and synthetic biology. Chromosoma (2016).
11. Jiang, W. et al. Cas9-Assisted Targeting of CHromosome segments CATCH enables one-step targeted cloning of large gene clusters. Nat. Commun. 6, 8101 (2015).
12. Bian, X., Plaza, A., Zhang, Y. & Müller, R. Luminmycins A-C, cryptic natural products from Photorhabdus luminescens identified by heterologous expression in Escherichia coli. J. Nat. Prod. 75, 1652-1655 (2012).
13. Zhou, Y. et al. Iterative mechanism of macrodiolide formation in the anticancer compound conglobatin. Chem. Biol. 22, 745-754 (2015).
14. te Riele, H., Maandag, E.R. & Berns, A. Highly efficient gene targeting in embryonic stem cells through homologous recombination with isogenic DNA constructs. Proc. Natl. Acad. Sci. USA 89, 5128-5132 (1992).
15. Baker, O. et al. RAC-tagging: Recombineering And Cas9-assisted targeting for protein tagging and conditional analyses. Sci. Rep. 6, 25529 (2016).
16. Fu, J., Teucher, M., Anastassiadis, K., Skarnes, W. & Stewart, A.F. A recombineering pipeline to make conditional targeting constructs. Methods Enzymol. 477, 125-144 (2010).
17. Hofemeister, H. et al. Recombineering, transfection, Western, IP and ChIP methods for protein tagging via gene targeting or BAC transgenesis. Methods 53, 437-452 (2011).
18. Copeland, N.G., Jenkins, N.A. & Court, D.L. Recombineering: a powerful new tool for mouse functional genomics. Nat. Rev. Genet. 2, 769-779 (2001).
19. Muyrers, J.P., Zhang, Y. & Stewart, A.F. Techniques: Recombinogenic engineering--new options for cloning and manipulating DNA. Trends Biochem. Sci. 26, 325-331 (2001).
20. Narayanan, K. & Chen, Q. Bacterial artificial chromosome mutagenesis using recombineering. J. Biomed. Biotechnol. 2011, 971296 (2011).
21. Murphy, K.C. Phage recombinases and their applications. Adv. Virus Res. 83, 367-414 (2012).
22. Murphy, K.C. lambda Recombination and Recombineering. EcoSal Plus 7 (2016).
23. Zhang, Y., Buchholz, F., Muyrers, J.P. & Stewart, A.F. A new logic for DNA engineering using recombination in Escherichia coli. Nat. Genet. 20, 123-128 (1998).
24. Clark, A.J. et al. Genes of the RecE and RecF pathways of conjugational recombination in Escherichia coli. Cold Spring Harb. Symp. Quant. Biol. 49, 453-462 (1984).
25. Hall, S.D., Kane, M.F. & Kolodner, R.D. Identification and characterization of the Escherichia coli RecT protein, a protein encoded by the recE region that promotes renaturation of homologous single-stranded DNA. J. Bacteriol. 175, 277-287 (1993).
26. Clark, A.J., Satin, L. & Chu, C.C. Transcription of the Escherichia coli recE gene from a promoter in Tn5 and IS50. J. Bacteriol. 176, 7024-7031 (1994).
27. Kuzminov, A. Recombinational repair of DNA damage in Escherichia coli and bacteriophage lambda. Microbiol. Mol. Biol. Rev. 63, 751-813 (1999).
28. Chu, C.C., Templin, A. & Clark, A.J. Suppression of a frameshift mutation in the recE gene of Escherichia coli K-12 occurs by gene fusion. J. Bacteriol. 171, 2101-2109 (1989).
29. Muyrers, J.P., Zhang, Y., Buchholz, F. & Stewart, A.F. RecE/RecT and Reda/Redb initiate double-stranded break repair by specifically interacting with their respective partners. Genes Dev. 14, 1971-1982 (2000).
30. Chang, H.W. & Julin, D.A. Structure and function of the Escherichia coli RecE protein, a member of the RecB nuclease domain family. J. Biol. Chem. 276, 46004-46010 (2001).
31. Zhang, J., Xing, X., Herr, A.B. & Bell, C.E. Crystal structure of E. coli RecE protein reveals a toroidal tetramer for processing double-stranded DNA breaks. Structure 17, 690-702 (2009).
32. Zhang, Y., Muyrers, J.P., Rientjes, J. & Stewart, A.F. Phage annealing proteins promote oligonucleotide-directed mutagenesis in Escherichia coli and mouse ES cells. BMC Mol. Biol. 4, 1 (2003).
33. Szczepanska, A.K. Bacteriophage-encoded functions engaged in initiation of homologous recombination events. Crit. Rev. Microbiol. 35, 197-220 (2009).
34. Kolodner, R., Hall, S.D. & Luisi-DeLuca, C. Homologous pairing proteins encoded by the Escherichia coli recE and recT genes. Mol Microbiol 11, 23-30 (1994).
35. Iyer, L.M., Koonin, E.V. & Aravind, L. Classification and evolutionary history of the single-strand annealing proteins, RecT, Redbeta, ERF and RAD52. BMC genomics 3, 8 (2002).
36. Maresca, M. et al. Single-stranded heteroduplex intermediates in lambda Red homologous recombination. BMC Mol. Biol. 11, 54 (2010).
37. Poteete, A.R. Involvement of Escherichia coli DNA replication proteins in phage Lambda Red-mediated homologous recombination. PloS one 8, e67440 (2013).
38. Muyrers, J.P., Zhang, Y., Testa, G. & Stewart, A.F. Rapid modification of bacterial artificial chromosomes by ET-recombination. Nucleic Acids Res. 27, 1555-1557 (1999).
39. Zhang, Y., Muyrers, J.P., Testa, G. & Stewart, A.F. DNA cloning by homologous recombination in Escherichia coli. Nat. Biotechnol. 18, 1314-1317 (2000).
40. Testa, G. et al. Engineering the mouse genome with bacterial artificial chromosomes to create multipurpose alleles. Nat. Biotechnol. 21, 443-447 (2003).
41. Sarov, M. et al. A recombineering pipeline for functional genomics applied to Caenorhabditis elegans. Nat. Methods 3, 839-844 (2006).
42. Bird, A.W. et al. High-efficiency counterselection recombineering for site-directed mutagenesis in bacterial artificial chromosomes. Nat. Methods 9, 103-109 (2012).
43. Yin, J. et al. Direct cloning and heterologous expression of the salinomycin biosynthetic gene cluster from Streptomyces albus DSM41398 in Streptomyces coelicolor A3(2). Sci. Rep. 5, 15081 (2015).
44. Wang, H. et al. Improved seamless mutagenesis by recombineering using ccdB for counterselection. Nucleic Acids Res 42, e37 (2014).
45. Pospiech, A. & Neumann, B. A versatile quick-prep of genomic DNA from Gram-positive bacteria. Trends Genet. 11, 217-218 (1995).
46. Wang, H. et al. RecET direct cloning and Redalphabeta recombineering of biosynthetic gene clusters, large operons or single genes for heterologous expression. Nat. Protoc. 11, 1175-1190 (2016).
47. Zhang, S.D. et al. Genome Sequence of Luminous Piezophile Photobacterium phosphoreum ANT-2200. Genome Announc. 2, e0009614 (2014).
48. Jinek, M. et al. A programmable dual-RNA-guided DNA endonuclease in adaptive bacterial immunity. Science 337, 816-821 (2012).
49. Gasiunas, G., Barrangou, R., Horvath, P. & Siksnys, V. Cas9-crRNA ribonucleoprotein complex mediates specific DNA cleavage for adaptive immunity in bacteria. Proc. Natl. Acad. Sci. USA 109, E2579-2586 (2012).
50. Skarnes, W.C. et al. A conditional knockout resource for the genome-wide study of mouse gene function. Nature 474, 337-342 (2011).
51. Torsvik, V., Goksoyr, J. & Daae, F.L. High diversity in DNA of soil bacteria. Appl. Environ. Microbiol. 56, 782-787 (1990).
52. Rappe, M.S. & Giovannoni, S.J. The uncultured microbial majority. Annu. Rev. Microbiol. 57, 369-394 (2003).
53. Charlop-Powers, Z., Milshteyn, A. & Brady, S.F. Metagenomic small molecule discovery methods. Curr. Opin. Microbiol. 19, 70-75 (2014).

## Claims

1. A method of homologous recombination, comprising:
treating two or more target nucleic acid molecules with a first exonuclease, and recombining the two or more target nucleic acid molecules in the presence of a second exonuclease and an annealing protein in a host cell, **wherein** the recombined target nucleic acid molecules share at least one identical sequence, wherein at least one identical sequence is inside or at one end of each of the two or more target nucleic acid molecules,
wherein said treating in the presence of the first exonuclease comprises in vitro joining; said in vitro joining comprising joining the two or more target nucleic acid molecules,
and wherein said treating in the presence of the first exonuclease comprises enzyme digestion and annealing;
and wherein the second exonuclease is RecE and the annealing protein is RecT.

2. The method of claim 1, **characterized in that** the at least one identical sequence comprises at least 6 nucleotides.

3. The method of claim 1, **characterized in that** the first exonuclease is selected from the group consisting of T4 DNA polymerase, Klenow fragment of DNA polymerase I, T5 exonuclease, and T7 exonuclease.

4. The method of claim 1, **characterized in that** treating two or more target nucleic acid molecules further comprises addition of a DNA polymerase, dNTPs and a DNA ligase.

5. The method of claim 1, **characterized in that** the host cell is a yeast cell or a bacterial cell and expresses an exonuclease and an annealing protein.

6. The method of claim 5, **characterized in that** the host cell is *E. coli* cell expressing full length RecE and RecT, or the host cell expresses the exonuclease and the annealing protein on a plasmid vector and/or a chromosome.

7. The method of claim 1, **characterized in that** the two or more target nucleic acid molecules are a linear DNA segment selected from a DNA fragment digested by endonuclease, a DNA fragment amplified by PCR, a genomic DNA fragment, a member of cDNA library, a fragment derived from bacterial artificial chromosomes (BACs) and a fragment of cloning vectors.

8. A method of assembling a nucleic acid molecule, comprising: treating two or more nucleic acid molecules with a first exonuclease, and recombining the two or more nucleic acid molecules in the presence of a second exonuclease and an annealing protein, wherein each nucleic acid molecule shares at least one identical sequence with an adjacent nucleic acid molecule in a resulting assembly product, and at least one identical sequence is inside or at one end of each of the two or more nucleic acid molecules, wherein said treating in the presence of the first exonuclease comprises in vitro joining; said in vitro joining comprising joining the two or more nucleic acid molecules, and wherein said treating in the presence of the first exonuclease comprises enzyme digestion and annealing; and wherein the second exonuclease is RecE and the annealing protein is RecT.

9. The method of claim 8, **characterized in that** the at least one identical sequence comprises at least 6 nucleotides.

10. The method of claim 8, **characterized in that** the first exonuclease is selected from the group consisting of T4 DNA polymerase, Klenow fragment of DNA polymerase I, T5 exonuclease, and T7 exonuclease.

11. The method of claim 8, **characterized in that** treating two or more nucleic acid molecules further comprises addition of a DNA polymerase, dNTPs and a DNA ligase.

12. The method of claim 8, **characterized in that** the host cell is a yeast cell or a bacterial cell and expresses an exonuclease and an annealing protein.

13. The method of claim 12, **characterized in that** the host cell is *E. coli* cell expressing full length RecE and RecT, or the host cell expresses the exonuclease and the annealing protein on a plasmid vector and/or a chromosome.

14. The method of claim 8, **characterized in that** the two or more nucleic acid molecules are a linear DNA segment selected from a DNA fragment digested by endonuclease, a DNA fragment amplified by PCR, a genomic DNA fragment, a member of cDNA library, a fragment derived from bacterial artificial chromosomes (BACs) and a fragment of cloning vectors.

15. A method of cloning a genomic DNA, comprising:
treating a linear cloning vector and a mixture of genomic DNA fragments with a first exonuclease, and recombining the linear cloning vector and a target DNA fragment of the mixture of genomic DNA fragments, **characterized in that** the linear cloning vector shares at least one identical sequence with the target DNA fragment of the mixture of genomic DNA fragments inside or at one end of each of the linear cloning vector and target DNA fragment,
wherein said treating in the presence of the first exonuclease comprises in vitro joining; said in vitro joining comprising joining the two or more nucleic acid molecules,
and wherein said treating in the presence of the first exonuclease comprises enzyme digestion and annealing;
and wherein the second exonuclease is RecE and the annealing protein is RecT.

16. The method of claim 15, **characterized in that** the at least one identical sequence comprises at least 6 nucleotides.

17. The method of claim 15, **characterized in that** the first exonuclease is selected from the group consisting of T4 DNA polymerase, Klenow fragment of DNA polymerase I, T5 exonuclease, and T7 exonuclease.

18. The method of claim 15, **characterized in that** treating a linear cloning vector and a mixture of genomic DNA fragments further comprises addition of a DNA polymerase, dNTPs and a DNA ligase.

19. The method of claim 15, **characterized in that** the host cell is a yeast cell or a bacterial cell and expresses an exonuclease and an annealing protein.

20. The method of claim 19, **characterized in that** the host cell is *E. coli* cell expressing full length RecE and RecT, or the host cell expresses the exonuclease and the annealing protein on a plasmid vector and/or a chromosome.

21. The method of claim 15, **characterized in that** the target DNA fragment is a linear DNA segment selected from a DNA fragment digested by endonuclease, a DNA fragment amplified by PCR, a genomic DNA fragment, a member of cDNA library, a fragment derived from bacterial artificial chromosomes (BACs), and a fragment of cloning vectors.

22. Use of a method of any one of claims 1-7 in DNA fragment assembly, construction of a targeting vector, or genotyping of mammalian cells.

23. Use of a method of any one of claims 8-14 in DNA fragment assembly.

24. Use of a method of any one of claims 15-21 in construction of a targeting vector, or genotyping of mammalian cells.

## Patentansprüche

1. Verfahren zur homologen Rekombination, umfassend:
Behandeln von zwei oder mehr Target-Nukleinsäuremolekülen mit einer ersten Exonuklease und Rekombinieren der zwei oder mehr Target-Nukleinsäuremoleküle in Gegenwart einer zweiten Exonuklease und eines Hybridisierungsproteins in einer Wirtszelle, wobei die rekombinierten Target-Nukleinsäuremoleküle mindestens eine identische Sequenz teilen, wobei mindestens eine identische Sequenz innerhalb oder an einem Ende von jedem der zwei oder mehr Target-Nukleinsäuremoleküle ist,
wobei die Behandlung in Gegenwart der ersten Exonuklease ein In-vitro-Verbinden umfasst, wobei das In-vitro-Verbinden das Verbinden der zwei oder mehr Target-Nukleinsäuremoleküle umfasst,
und wobei die Behandlung in Anwesenheit der ersten Exonuklease Enzymverdauung und Hybridisierung umfasst;
und wobei die zweite Exonuklease RecE ist und das Hybridisierungsprotein RecT ist.

2. Verfahren nach Anspruch 1, **dadurch gekennzeichnet, dass** die mindestens eine identische Sequenz mindestens 6 Nukleotide umfasst.

3. Verfahren nach Anspruch 1, **dadurch gekennzeichnet, dass** die erste Exonuklease ausgewählt ist aus der Gruppe bestehend aus T4-DNA-Polymerase, Klenow-Fragment der DNA-Polymerase I, T5-Exonuklease und T7-Exonuklease.

4. Verfahren nach Anspruch 1, **dadurch gekennzeichnet, dass** die Behandlung von zwei oder mehr Target-Nukleinsäuremolekülen weiterhin die Zugabe einer DNA-Polymerase, dNTPs und einer DNA-Ligase umfasst.

5. Verfahren nach Anspruch 1, **dadurch gekennzeichnet, dass** die Wirtszelle eine Hefezelle oder eine Bakterienzelle ist und eine Exonuklease und ein Hybridisierungsprotein exprimiert.

6. Verfahren nach Anspruch 5, **dadurch gekennzeichnet, dass** die Wirtszelle eine E. *coli*-Zelle ist, die RecE und RecT in voller Länge exprimiert, oder die Wirtszelle die Exonuklease und das Hybridisierungsprotein auf einem Plasmidvektor und/oder einem Chromosom exprimiert.

7. Verfahren nach Anspruch 1, **dadurch gekennzeichnet, dass** die zwei oder mehr Target-Nukleinsäuremoleküle ein lineares DNA-Segment sind, das ausgewählt ist aus einem DNA-Fragment, das durch eine Endonuklease verdaut wurde, einem DNA-Fragment, das durch PCR amplifiziert wurde, einem genomischen DNA-Fragment, einem Mitglied einer cDNA-Datenbank, einem Fragment, das von bakteriellen künstlichen Chromosomen (BACs "bacterial artificial chromosomes") stammt, und einem Fragment von Klonierungsvektoren.

8. Verfahren zum Zusammenbau eines Nukleinsäuremoleküls, umfassend: Behandeln von zwei oder mehr Nukleinsäuremolekülen mit einer ersten Exonuklease und Rekombinieren der zwei oder mehr Target-Nukleinsäuremoleküle in Gegenwart einer zweiten Exonuklease und eines Hybridisierungsproteins, wobei jedes Nukleinsäuremolekül mindestens eine identische Sequenz mit einem benachbarten Nukleinsäuremolekül in einem resultierenden Konstrukt teilt und mindestens eine identische Sequenz innerhalb oder an einem Ende von jedem der zwei oder mehr Nukleinsäuremoleküle ist,
wobei die Behandlung in Gegenwart der ersten Exonuklease ein in vitro-Verbinden umfasst, wobei das in vitro-Verbinden das Verbinden der zwei oder mehr Nukleinsäuremoleküle umfasst,
und wobei die Behandlung in Anwesenheit der ersten Exonuklease Enzymverdauung und Hybridisierung umfasst;
und wobei die zweite Exonuklease RecE ist und das Hybridisierungsprotein RecT ist.

9. Verfahren nach Anspruch 8, **dadurch gekennzeichnet, dass** die mindestens eine identische Sequenz mindestens 6 Nukleotide umfasst.

10. Verfahren nach Anspruch 8, **dadurch gekennzeichnet, dass** die erste Exonuklease ausgewählt ist aus der Gruppe bestehend aus T4-DNA-Polymerase, Klenow-Fragment der DNA-Polymerase I, T5-Exonuklease und T7-Exonuklease.

11. Verfahren nach Anspruch 8, **dadurch gekennzeichnet, dass** die Behandlung von zwei oder mehr Nukleinsäuremolekülen ferner die Zugabe einer DNA-Polymerase, dNTPs und einer DNA-Ligase umfasst.

12. Verfahren nach Anspruch 8, **dadurch gekennzeichnet, dass** die Wirtszelle eine Hefezelle oder eine Bakterienzelle ist und eine Exonuklease und ein Hybridisierungsprotein exprimiert.

13. Verfahren nach Anspruch 12, **dadurch gekennzeichnet, dass** die Wirtszelle eine *E*. *coli*-Zelle ist, die RecE und RecT in voller Länge exprimiert, oder die Wirtszelle die Exonuklease und das Hybridisierungsprotein auf einem Plasmidvektor und/oder einem Chromosom exprimiert.

14. Verfahren nach Anspruch 8, **dadurch gekennzeichnet, dass** die zwei oder mehr Nukleinsäuremoleküle ein lineares DNA-Segment sind, das ausgewählt ist aus einem DNA-Fragment, das durch eine Endonuklease verdaut wurde, einem DNA-Fragment, das durch PCR amplifiziert wurde, einem genomischen DNA-Fragment, einem Mitglied einer cDNA-Datenbank, einem Fragment, das von bakteriellen künstlichen Chromosomen (BACs) stammt, und einem Fragment von Klonierungsvektoren.

15. Verfahren zum Klonieren einer genomischen DNA, umfassend: Behandeln eines linearen Klonierungsvektors und eines Gemischs genomischer DNA-Fragmente mit einer ersten Exonuklease, und Rekombinieren des linearen Klonierungsvektors und eines Target-DNA-Fragments des Gemischs genomischer DNA-Fragmente, **dadurch gekennzeichnet, dass** der lineare Klonierungsvektor mindestens eine identische Sequenz mit dem Target-DNA-Fragment des Gemischs genomischer DNA-Fragmente innerhalb oder an einem Ende von jedem des linearen Klonierungsvektors und des Target-DNA-Fragments teilt,
wobei die Behandlung in Gegenwart der ersten Exonuklease eine In-vitro-Verbinden umfasst, wobei das In-vitro-Verbinden das Verbinden der zwei oder mehr Nukleinsäuremoleküle umfasst,
und wobei die Behandlung in Anwesenheit der ersten Exonuklease Enzymverdauung und Hybridisierung umfasst;
und wobei die zweite Exonuklease RecE ist und das Hybridisierungsprotein RecT ist.

16. Verfahren nach Anspruch 15, **dadurch gekennzeichnet, dass** die mindestens eine identische Sequenz mindestens 6 Nukleotide umfasst.

17. Verfahren nach Anspruch 15, **dadurch gekennzeichnet, dass** die erste Exonuklease ausgewählt ist aus der Gruppe bestehend aus T4-DNA-Polymerase, Klenow-Fragment der DNA-Polymerase I, T5-Exonuklease und T7-Exonuklease.

18. Verfahren nach Anspruch 15, **dadurch gekennzeichnet, dass** die Behandlung eines linearen Klonierungsvektors und einer Mischung genomischer DNA-Fragmente ferner die Zugabe einer DNA-Polymerase, dNTPs und einer DNA-Ligase umfasst.

19. Verfahren nach Anspruch 15, **dadurch gekennzeichnet, dass** die Wirtszelle eine Hefezelle oder eine Bakterienzelle ist und eine Exonuklease und ein Hybridisierungsprotein exprimiert.

20. Verfahren nach Anspruch 19, **dadurch gekennzeichnet, dass** die Wirtszelle eine E. *coli*-Zelle ist, die RecE und RecT in voller Länge exprimiert, oder die Wirtszelle die Exonuklease und das Hybridisierungsprotein auf einem Plasmidvektor und/oder einem Chromosom exprimiert.

21. Verfahren nach Anspruch 15, **dadurch gekennzeichnet, dass** das Target-DNA-Fragment ein lineares DNA-Segment ist, das ausgewählt ist aus einem DNA-Fragment, das durch eine Endonuklease verdaut wurde, einem DNA-Fragment, das durch PCR amplifiziert wurde, einem genomischen DNA-Fragment, einem Bestandteil einer cDNA-Datenbank, einem Fragment, das von bakteriellen künstlichen Chromosomen (BACs) stammt, und einem Fragment von Klonierungsvektoren.

22. Verwendung eines Verfahrens nach einem der Ansprüche 1-7 bei dem DNA-Fragment-Zusammenbau, der Konstruktion eines Targeting-Vektors oder der Genotypisierung von Säugetierzellen.

23. Verwendung eines Verfahrens nach einem der Ansprüche 8-14 beim Zusammenbau von DNA-Fragmenten.

24. Verwendung eines Verfahrens nach einem der Ansprüche 15-21 bei der Konstruktion eines Targeting-Vektors oder bei der Genotypisierung von Säugetierzellen.

## Revendications

1. Procédé de recombinaison homologue, comprenant :
le traitement d'au moins deux molécules d'acide nucléique cibles par une première exonucléase, et la recombinaison desdites au moins deux molécules d'acide nucléique cibles en présence d'une seconde exonucléase et d'une protéine d'hybridation dans une cellule hôte, dans lequel les molécules d'acide nucléique cibles recombinées partagent au moins une séquence identique, au moins une séquence identique étant à l'intérieur ou à une extrémité de chacune desdites au moins deux molécules d'acide nucléique cibles,
dans lequel ledit traitement en présence de la première exonucléase comprend une jonction in vitro ; ladite jonction in vitro comprenant la jonction desdites au moins deux molécules d'acide nucléique cibles, et
dans lequel ledit traitement en présence de la première exonucléase comprend une digestion enzymatique et une hybridation ; et
dans lequel la seconde exonucléase est RecE et la protéine d'hybridation est RecT.

2. Procédé selon la revendication 1, **caractérisé par le fait que** ladite au moins une séquence identique comprend au moins 6 nucléotides.

3. Procédé selon la revendication 1, **caractérisé par le fait que** ladite première exonucléase est choisie dans le groupe consistant en l'ADN polymérase T4, le fragment de Klenow de l'ADN polymérase I, l'exonucléase T5 et l'exonucléase T7.

4. Procédé selon la revendication 1, **caractérisé par le fait que** le traitement d'au moins deux molécules d'acide nucléique cibles comprend en outre l'addition d'une ADN polymérase, de dNTPs et d'une ADN ligase.

5. Procédé selon la revendication 1, **caractérisé par le fait que** la cellule hôte est une cellule de levure ou une cellule bactérienne et exprime une exonucléase et une protéine d'hybridation.

6. Procédé selon la revendication 5, **caractérisé par le fait que** la cellule hôte est une cellule d'*E*. *coli* exprimant les RecE et RecT de longueur totale, ou la cellule hôte exprime l'exonucléase et la protéine d'hybridation sur un vecteur plasmidique et/ou un chromosome.

7. Procédé selon la revendication 1, **caractérisé par le fait que** lesdites au moins deux molécules d'acide nucléique cibles sont un segment d'ADN linéaire choisi parmi un fragment d'ADN digéré par endonucléase, un fragment d'ADN amplifié par PCR, un fragment d'ADN génomique, un membre de banque d'ADNc, un fragment dérivé de chromosomes artificiels bactériens (BACs) et un fragment de vecteurs de clonage.

8. Procédé d'assemblage d'une molécule d'acide nucléique, comprenant : le traitement d'au moins deux molécules d'acide nucléique par une première exonucléase, et la recombinaison desdites au moins deux molécules d'acide nucléique en présence d'une seconde exonucléase et d'une protéine d'hybridation, chaque molécule d'acide nucléique partageant au moins une séquence identique avec une molécule d'acide nucléique adjacente dans un produit d'assemblage résultant, et au moins une séquence identique étant à l'intérieur ou à une extrémité de chacune desdites au moins deux molécules d'acide nucléique,
dans lequel ledit traitement en présence de la première exonucléase comprend une jonction in vitro ; ladite jonction in vitro comprenant la jonction desdites au moins deux molécules d'acide nucléique, et
dans lequel ledit traitement en présence de la première exonucléase comprend une digestion enzymatique et une hybridation ; et
dans lequel la seconde exonucléase est RecE et la protéine d'hybridation est RecT.

9. Procédé selon la revendication 8, **caractérisé par le fait que** ladite au moins une séquence identique comprend au moins 6 nucléotides.

10. Procédé selon la revendication 8, **caractérisé par le fait que** la première exonucléase est choisie dans le groupe consistant en l'ADN polymérase T4, le fragment de Klenow de l'ADN polymérase I, l'exonucléase T5 et l'exonucléase T7.

11. Procédé selon la revendication 8, **caractérisé par le fait que** le traitement d'au moins deux molécules d'acide nucléique comprend en outre l'addition d'une ADN polymérase, de dNTPs et d'une ADN ligase.

12. Procédé selon la revendication 8, **caractérisé par le fait que** la cellule hôte est une cellule de levure ou une cellule bactérienne et exprime une exonucléase et une protéine d'hybridation.

13. Procédé selon la revendication 12, **caractérisé par le fait que** la cellule hôte est une cellule *d'E. coli* exprimant les RecE et RecT de longueur totale, ou la cellule hôte exprime l'exonucléase et la protéine d'hybridation sur un vecteur plasmidique et/ou un chromosome.

14. Procédé selon la revendication 8, **caractérisé par le fait que** lesdites au moins deux molécules d'acide nucléique sont un segment d'ADN linéaire choisi parmi un fragment d'ADN digéré par endonucléase, un fragment d'ADN amplifié par PCR, un fragment d'ADN génomique, un membre de banque d'ADNc, un fragment dérivé de chromosomes artificiels bactériens (BACs) et un fragment de vecteurs de clonage.

15. Procédé de clonage d'un ADN génomique, comprenant : le traitement d'un vecteur de clonage linéaire et d'un mélange de fragments d'ADN génomique avec une première exonucléase, et la recombinaison du vecteur de clonage linéaire et d'un fragment d'ADN cible du mélange de fragments d'ADN génomique, **caractérisé par le fait que** le vecteur de clonage linéaire partage au moins une séquence identique avec le fragment d'ADN cible du mélange de fragments d'ADN génomique à l'intérieur ou à une extrémité de chacun du vecteur de clonage linéaire et du fragment d'ADN cible,
dans lequel ledit traitement en présence de la première exonucléase comprend une jonction in vitro ; ladite jonction in vitro comprenant la jonction desdits au moins deux molécules d'acide nucléique, et
dans lequel ledit traitement en présence de la première exonucléase comprend une digestion enzymatique et une hybridation ; et
dans lequel la seconde exonucléase est RecE et la protéine d'hybridation est RecT.

16. Procédé selon la revendication 15, **caractérisé par le fait que** ladite au moins une séquence identique comprend au moins 6 nucléotides.

17. Procédé selon la revendication 15, **caractérisé par le fait que** la première exonucléase est choisie dans le groupe consistant en l'ADN polymérase T4, le fragment de Klenow de l'ADN polymérase I, l'exonucléase T5 et l'exonucléase T7.

18. Procédé selon la revendication 15, **caractérisé par le fait que** le traitement d'un vecteur de clonage linéaire et d'un mélange de fragments d'ADN génomique comprend en outre l'addition d'une ADN polymérase, de dNTPs et d'une ADN ligase.

19. Procédé selon la revendication 15, **caractérisé par le fait que** la cellule hôte est une cellule de levure ou une cellule bactérienne et exprime une exonucléase et une protéine d'hybridation.

20. Procédé selon la revendication 19, **caractérisé par le fait que** la cellule hôte est une cellule d'*E. coli* exprimant les RecE et RecT de longueur totale, ou la cellule hôte exprime l'exonucléase et la protéine d'hybridation sur un vecteur plasmidique et/ou un chromosome.

21. Procédé selon la revendication 15, **caractérisé par le fait que** le fragment d'ADN cible est un segment d'ADN linéaire choisi parmi un fragment d'ADN digéré par endonucléase, un fragment d'ADN amplifié par PCR, un fragment d'ADN génomique, un membre de banque d'ADNc, un fragment dérivé de chromosomes artificiels bactériens (BACs) et un fragment de vecteurs de clonage.

22. Utilisation d'un procédé selon l'une quelconque des revendications 1 à 7 dans l'assemblage de fragments d'ADN, la construction d'un vecteur de ciblage ou le génotypage de cellules de mammifères.

23. Utilisation d'un procédé selon l'une quelconque des revendications 8 à 14 dans l'assemblage de fragments d'ADN.

24. Utilisation d'un procédé selon l'une quelconque des revendications 15 à 21 dans la construction d'un vecteur de ciblage ou le génotypage de cellules de mammifères.
